# EUROPEAN PATENT APPLICATION

(11) **EP 3 269 814 A1**
(43) Date of publication of application: **17.01.2018**
(21) Application number: 16761767.9
(22) Date of filing: 08.03.2016
(51) Int. Cl.: C12N 15/113, C12P 21/02

(54) **TRANSLATION ENHANCER FOR USE IN CELL-FREE PROTEIN SYNTHESIS SYSTEM AND USE THEREOF**

(30) Priority: 09.03.2015 JP 2015046470
(71) Applicant: National University Corporation Nagoya University, Nagoya-shi, Aichi 464-8601 (JP)
(72) Inventor: TADA Yasuomi, Nagoya-shi Aichi 464-8601 (JP); NOMOTO Mika, Nagoya-shi Aichi 464-8601 (JP)
(74) Representative: Heyerhoff Geiger & Partner Patentanwälte PartGmbB
(86) International application number: PCT/JP2016/057249
(87) International publication number: WO 2016/143799

(57) **Abstract**

This specification provides a translation enhancer that makes it possible to obtain translation template mRNA efficiently in a cell-free protein synthesis system and, in turn, to realize excellent translation efficiency. Therefore, in this specification, a nucleic acid of at most 200 bases in length as a 3' untranslated region linked adjacent to the 3' end of a coding region that encodes the amino acid sequence of a desired protein, is used as a translation enhancer in a cell-free protein synthesis system.

SEQUENCE LISTING

<110>  Nattional University Corporatin Nagoya University

<120>  Non-Cellular Protein Synthesis System

<130>  K14-382-PCT

<160>  74

<170>  PatentIn version 3.5

<210>  1
<211>  1200
<212>  DNA
<213>  Artificial Sequence

<220>
<223>  Artificial 3'UTR

<400>  1
aaaaaaaaaa aaaaaagagc tcttggatcc ggccataagg gcctgatcct tcgagggggg      60

gcccggtacc agcttttgtt ccctttagtg agggttaatt tcgagcttgg cgtaatcatg     120

gtcatagctg tttcctgtgt gaaattgtta tccgctcaca attccacaca acatacgagc     180

cggaagcata aagtgtaaag cctggggtgc ctaatgagtg agctaactca cattaattgc     240

gttgcgctca ctgcccgctt tccagtcggg aaacctgtcg tgccagctgc attaatgaat     300

cggccaacgc gcggggagag gcggtttgcg tattgggcgc tcttccgctt cctcgctcac     360

tgactcgctg cgctcggtcg ttcggctgcg gcgagcggta tcagctcact caaaggcggt     420

aatacggtta tccacagaat caggggataa cgcaggaaag aacatgtgag caaaaggcca     480

gcaaaaggcc aggaaccgta aaaaggccgc gttgctggcg tttttccata ggctccgccc     540

ccctgacgag catcacaaaa atcgacgctc aagtcagagg tggcgaaacc cgacaggact     600

ataaagatac caggcgtttc cccctggaag ctccctcgtg cgctctcctg ttccgaccct     660

gccgcttacc ggatacctgt ccgcctttct cccttcggga agcgtggcgc tttctcatag     720

ctcacgctgt aggtatctca gttcggtgta ggtcgttcgc tccaagctgg gctgtgtgca     780

cgaacccccc gttcagcccg accgctgcgc cttatccggt aactatcgtc ttgagtccaa     840

cccggtaaga cacgacttat cgccactggc agcagccact ggtaacagga ttagcagagc     900

gaggtatgta ggcggtgcta cagagttctt gaagtggtgg cctaactacg gctacactag     960

aagaacagta tttggtatct gcgctctgct gaagccagtt accttcggaa aaagagttgg    1020

tagctcttga tccggcaaac aaaccaccgc tggtagcggt ggtttttttg tttgcaagca    1080

gcagattacg cgcagaaaaa aaggatctca agaagatcct ttgatctttt ctacggggtc    1140

tgacgctcag tggaacgaaa actcacgtta agggattttg gtcatgagat tatcaaaaag    1200

<210>  2

<211>  600
<212>  DNA
<213>  Artificial Sequence

<220>
<223>  Artificial 3'UTR

<400>  2
aaaaaaaaaa aaaaaagagc tcttggatcc ggccataagg gcctgatcct tcgagggggg      60

gcccggtacc agcttttgtt ccctttagtg agggttaatt tcgagcttgg cgtaatcatg     120

gtcatagctg tttcctgtgt gaaattgtta tccgctcaca attccacaca acatacgagc     180

cggaagcata aagtgtaaag cctggggtgc ctaatgagtg agctaactca cattaattgc     240

gttgcgctca ctgcccgctt tccagtcggg aaacctgtcg tgccagctgc attaatgaat     300

cggccaacgc gcggggagag gcggtttgcg tattgggcgc tcttccgctt cctcgctcac     360

tgactcgctg cgctcggtcg ttcggctgcg gcgagcggta tcagctcact caaaggcggt     420

aatacggtta tccacagaat caggggataa cgcaggaaag aacatgtgag caaaaggcca     480

gcaaaaggcc aggaaccgta aaaaggccgc gttgctggcg tttttccata ggctccgccc     540

ccctgacgag catcacaaaa atcgacgctc aagtcagagg tggcgaaacc cgacaggact     600


<210>  3
<211>  300
<212>  DNA
<213>  Artificial Sequence

<220>
<223>  Artificial 3'UTR

<400>  3
aaaaaaaaaa aaaaaagagc tcttggatcc ggccataagg gcctgatcct tcgagggggg      60

gcccggtacc agcttttgtt ccctttagtg agggttaatt tcgagcttgg cgtaatcatg     120

gtcatagctg tttcctgtgt gaaattgtta tccgctcaca attccacaca acatacgagc     180

cggaagcata aagtgtaaag cctggggtgc ctaatgagtg agctaactca cattaattgc     240

gttgcgctca ctgcccgctt tccagtcggg aaacctgtcg tgccagctgc attaatgaat     300


<210>  4
<211>  100
<212>  DNA
<213>  Artificial Sequence

<220>
<223>  Artificial 3'UTR

<400>  4
aaaaaaaaaa aaaaaagagc tcttggatcc ggccataagg gcctgatcct tcgagggggg      60

gcccggtacc agcttttgtt ccctttagtg agggttaatt                           100

<210>  5
<211>  50
<212>  DNA
<213>  Artificial Sequence

<220>
<223>  Artificial 3'UTR

<400>  5
aaaaaaaaaa aaaaaagagc tcttggatcc ggccataagg gcctgatcct                50


<210>  6
<211>  40
<212>  DNA
<213>  Artificial Sequence

<220>
<223>  Artificial 3'UTR

<400>  6
aaaaaaaaaa aaaaaagagc tcttggatcc ggccataagg                           40


<210>  7
<211>  34
<212>  DNA
<213>  Artificial Sequence

<220>
<223>  Primer

<400>  7
ccagcaggga ggtactatgg acggttcttc gttt                                 34


<210>  8
<211>  28
<212>  DNA
<213>  Artificial Sequence

<220>
<223>  Primer

<400>  8
cccgcgaaat taatacgact cactatag                                        28


<210>  9
<211>  119
<212>  DNA
<213>  Artificial Sequence

<220>
<223>  Primer

<400>  9
cgactcacta tagggctcac ctatctctct acacaaaaca tttccctaca tacaactttc      60

aacttcctat tatggactac aaggatgacg atgacaagct ccagcaggga ggtactatg      119


<210>  10

<211>  21
<212>  DNA
<213>  Artificial Sequence

<220>
<223>  Primer

<400>  10
ctttttgata atctcatgac c                                                21


<210>  11
<211>  17
<212>  DNA
<213>  Artificial Sequence

<220>
<223>  Primer

<400>  11
agtcctgtcg ggtttcg                                                     17


<210>  12
<211>  19
<212>  DNA
<213>  Artificial Sequence

<220>
<223>  Primer

<400>  12
attcattaat gcagctggc                                                   19


<210>  13
<211>  19
<212>  DNA
<213>  Artificial Sequence

<220>
<223>  Primer

<400>  13
aattaaccct cactaaagg                                                   19


<210>  14
<211>  17
<212>  DNA
<213>  Artificial Sequence

<220>
<223>  Primer

<400>  14
aggatcaggc ccttatg                                                     17


<210>  15
<211>  16
<212>  DNA
<213>  Artificial Sequence

<220>
<223>  Primer

<400>  15
ccttatggcc ggatcc                                                        16


<210>  16
<211>  34
<212>  DNA
<213>  Artificial Sequence

<220>
<223>  Primer

<400>  16
ccagcaggga ggtactatgg tttccaacat tgat                                    34


<210>  17
<211>  34
<212>  DNA
<213>  Artificial Sequence

<220>
<223>  Primer

<400>  17
ccagcaggga ggtactatgg gtgtaacatt agaa                                    34


<210>  18
<211>  21
<212>  DNA
<213>  Artificial Sequence

<220>
<223>  Primer

<400>  18
gggatatcag ctggatggca a                                                  21


<210>  19
<211>  55
<212>  DNA
<213>  Artificial Sequence

<220>
<223>  Primer

<400>  19
ccttatggcc ggatccaaga gctctttttt ttttttaatc aaaagcagaa atgtt             55


<210>  20
<211>  55
<212>  DNA
<213>  Artificial Sequence

<220>
<223>  Primer

<400>  20
ccttatggcc ggatccaaga gctctttttt ttttttaaac acagttagtt tcggt          55


<210>  21
<211>  19
<212>  DNA
<213>  Artificial Sequence

<220>
<223>  Primer

<400>  21
gggatatcag ctggatggc                                                  19


<210>  22
<211>  20
<212>  DNA
<213>  Artificial Sequence

<220>
<223>  Primer

<400>  22
agtgacctgt tcgttgcaac                                                 20


<210>  23
<211>  16
<212>  DNA
<213>  Artificial Sequence

<220>
<223>  Primer

<400>  23
ggccccccct cgaagg                                                     16


<210>  24
<211>  35
<212>  DNA
<213>  Artificial Sequence

<220>
<223>  Primer

<400>  24
ccctcgaagg atcaggccct tatggccgga tccaa                                35


<210>  25
<211>  20
<212>  DNA
<213>  Artificial Sequence

<220>
<223>  Primer

<400>  25
gactgatagt gacctgttcg                                                 20

<210>  26
<211>  37
<212>  DNA
<213>  Artificial Sequence

<220>
<223>  Artificial 3'UTR

<400>  26
aaaaaaaaaa gagctcttgg atccggccat aagggcc                                37


<210>  27
<211>  34
<212>  DNA
<213>  Artificial Sequence

<220>
<223>  Artificial 3'UTR

<400>  27
ccagcaggga ggtactatga acagaggaag ctta                                   34


<210>  28
<211>  34
<212>  DNA
<213>  Artificial Sequence

<220>
<223>  Primer

<400>  28
ccagcaggga ggtactatga agaaaggtgg attc                                   34


<210>  29
<211>  34
<212>  DNA
<213>  Artificial Sequence

<220>
<223>  Primer

<400>  29
ccagcaggga ggtactatga gaccgattca atcg                                   34


<210>  30
<211>  34
<212>  DNA
<213>  Artificial Sequence

<220>
<223>  Primer

<400>  30
ccagcaggga ggtactatga aaccgattca atct                                   34


<210>  31

<211>  34
<212>  DNA
<213>  Artificial Sequence

<220>
<223>  Primer

<400>  31
ccagcaggga ggtactatgg tgaagatcaa atcg                                34


<210>  32
<211>  34
<212>  DNA
<213>  Artificial Sequence

<220>
<223>  Primer

<400>  32
ccagcaggga ggtactatgg ctttagtacg tgaa                                34


<210>  33
<211>  55
<212>  DNA
<213>  Artificial Sequence

<220>
<223>  Primer

<400>  33
ccttatggcc ggatccaaga gctctttttt ttttttagtt agcaagtggg ggaat          55


<210>  34
<211>  55
<212>  DNA
<213>  Artificial Sequence

<220>
<223>  Primer

<400>  34
ccttatggcc ggatccaaga gctctttttt ttttttacac ggagaacgta ccaga          55


<210>  35
<211>  55
<212>  DNA
<213>  Artificial Sequence

<220>
<223>  Primer

<400>  35
ccttatggcc ggatccaaga gctctttttt ttttttatgt ggttggagaa gaaga          55


<210>  36
<211>  55
<212>  DNA
<213>  Artificial Sequence

<220>
<223>  Primer

<400>  36
ccttatggcc ggatccaaga gctctttttt ttttttaaga ggcagaagga agagg          55


<210>  37
<211>  55
<212>  DNA
<213>  Artificial Sequence

<220>
<223>  Primer

<400>  37
ccttatggcc ggatccaaga gctctttttt ttttttatct aaggtagtta acagg          55


<210>  38
<211>  55
<212>  DNA
<213>  Artificial Sequence

<220>
<223>  Primer

<400>  38
ccttatggcc ggatccaaga gctctttttt ttttttaaag atcttcccgg agaaa          55


<210>  39
<211>  58
<212>  DNA
<213>  Artificial Sequence

<220>
<223>  Primer

<400>  39
cacaaaacat ttccctacat acaactttca acttcctatt atggatccat ttttaatt          58


<210>  40
<211>  58
<212>  DNA
<213>  Artificial Sequence

<220>
<223>  Primer

<400>  40
cacaaaacat ttccctacat acaactttca acttcctatt atggacggtt cttcgttt          58


<210>  41
<211>  58
<212>  DNA
<213>  Artificial Sequence

<220>
<223>  Primer

<400>  41
cacaaaacat ttccctacat acaactttca acttcctatt atggctgata ccagtccg          58


<210>  42
<211>  58
<212>  DNA
<213>  Artificial Sequence

<220>
<223>  Primer

<400>  42
cacaaaacat ttccctacat acaactttca acttcctatt atggacacca ccattgat          58


<210>  43
<211>  58
<212>  DNA
<213>  Artificial Sequence

<220>
<223>  Primer

<400>  43
cacaaaacat ttccctacat acaactttca acttcctatt atgactgatt accggcta          58


<210>  44
<211>  58
<212>  DNA
<213>  Artificial Sequence

<220>
<223>  Primer

<400>  44
cacaaaacat ttccctacat acaactttca acttcctatt atggtttccg gagtcggg          58


<210>  45
<211>  39
<212>  DNA
<213>  Artificial Sequence

<220>
<223>  Primer

<400>  45
agtacctccc tgctggagac cccaagtccc actattttc                               39


<210>  46
<211>  39
<212>  DNA
<213>  Artificial Sequence

<220>
<223>  Primer

<400>  46
agtacctccc tgctggagac ctgttctaga ttgctccat                               39

<210>  47
<211>  39
<212>  DNA
<213>  Artificial Sequence

<220>
<223>  Primer

<400>  47
agtacctccc tgctggagac cctctctggg tcgagcaag                              39


<210>  48
<211>  39
<212>  DNA
<213>  Artificial Sequence

<220>
<223>  Primer

<400>  48
agtacctccc tgctggagac cccgacgacg atgagagag                              39


<210>  49
<211>  39
<212>  DNA
<213>  Artificial Sequence

<220>
<223>  Primer

<400>  49
agtacctccc tgctggagac caccgatttt tgaaatcaa                              39


<210>  50
<211>  39
<212>  DNA
<213>  Artificial Sequence

<220>
<223>  Primer

<400>  50
agtacctccc tgctggagac catatggcat catcagcat                              39


<210>  51
<211>  60
<212>  DNA
<213>  Artificial Sequence

<220>
<223>  Primer

<400>  51
cccgcgaaat taatacgact cactataggg ctcacctatc tctctacaca aaacatttcc      60


<210>  52

<211>  94
<212>  DNA
<213>  Artificial Sequence

<220>
<223>  Primer

<400>  52
ccctcgaagg atcaggccct tatggccgga tccaagagct cttttttttt tttacttgtc      60

atcgtcatcc ttgtagtcag tacctccctg ctgg                                  94


<210>  53
<211>  34
<212>  DNA
<213>  Artificial Sequence

<220>
<223>  Primer

<400>  53
ccagcaggga ggtactatgg acggttcttc gttt                                  34


<210>  54
<211>  34
<212>  DNA
<213>  Artificial Sequence

<220>
<223>  Primer

<400>  54
ccagcaggga ggtactatgg acggttcttc gttt                                  34


<210>  55
<211>  34
<212>  DNA
<213>  Artificial Sequence

<220>
<223>  Primer

<400>  55
ccagcaggga ggtactactg aaacatcgga caca                                  34


<210>  56
<211>  34
<212>  DNA
<213>  Artificial Sequence

<220>
<223>  Primer

<400>  56
ccagcaggga ggtactatga atgcttctga aggg                                  34


<210>  57
<211>  55

<212>  DNA
<213>  Artificial Sequence

<220>
<223>  Primer

<400>  57
ccttatggcc ggatccaaga gctctttttt ttttttatgt tctagattgc tccat          55


<210>  58
<211>  52
<212>  DNA
<213>  Artificial Sequence

<220>
<223>  Primer

<400>  58
ccttatggcc ggatccaaga gctctttttt ttttttacaa gcttgtgtcc ga             52


<210>  59
<211>  52
<212>  DNA
<213>  Artificial Sequence

<220>
<223>  Primer

<400>  59
ccttatggcc ggatccaaga gctctttttt ttttttatgt agcatcccct tc             52


<210>  60
<211>  55
<212>  DNA
<213>  Artificial Sequence

<220>
<223>  Primer

<400>  60
ccttatggcc ggatccaaga gctctttttt ttttttatgt tctagattgc tccat          55


<210>  61
<211>  34
<212>  DNA
<213>  Artificial Sequence

<220>
<223>  Primer

<400>  61
ccagcaggga ggtactatgg acaccaccat tgat                                 34


<210>  62
<211>  34
<212>  DNA
<213>  Artificial Sequence

<220>
<223>  Primer

<400>  62
ccagcaggga ggtactatgg gaactcacat caat                                    34


<210>  63
<211>  26
<212>  DNA
<213>  Artificial Sequence

<220>
<223>  Primer

<400>  63
tttttttacc gacgacgatg agagag                                             26


<210>  64
<211>  31
<212>  DNA
<213>  Artificial Sequence

<220>
<223>  Primer

<400>  64
tttttttttt ttaccgacga cgatgagaga g                                       31


<210>  65
<211>  41
<212>  DNA
<213>  Artificial Sequence

<220>
<223>  Primer

<400>  65
tttttttttt tttttttttt ttaccgacga cgatgagaga g                            41


<210>  66
<211>  51
<212>  DNA
<213>  Artificial Sequence

<220>
<223>  Primer

<400>  66
tttttttttt tttttttttt tttttttttt ttaccgacga cgatgagaga g                 51


<210>  67
<211>  61
<212>  DNA
<213>  Artificial Sequence

<220>
<223>  Primer

<400>  67
tttttttttt tttttttttt tttttttttt tttttttttt ttaccgacga cgatgagaga      60

g                                                                      61


<210>  68
<211>  26
<212>  DNA
<213>  Artificial Sequence

<220>
<223>  Primer

<400>  68
tttttttacc atggtccggt taatgt                                           26


<210>  69
<211>  31
<212>  DNA
<213>  Artificial Sequence

<220>
<223>  Primer

<400>  69
tttttttttt ttaccatggt ccggttaatg t                                     31


<210>  70
<211>  41
<212>  DNA
<213>  Artificial Sequence

<220>
<223>  Primer

<400>  70
tttttttttt tttttttttt ttaccatggt ccggttaatg t                          41


<210>  71
<211>  51
<212>  DNA
<213>  Artificial Sequence

<220>
<223>  Primer

<400>  71
tttttttttt tttttttttt tttttttttt ttaccatggt ccggttaatg t               51


<210>  72
<211>  61
<212>  DNA
<213>  Artificial Sequence

<220>
<223>  Primer

<400>  72

tttttttttt tttttttttt tttttttttt tttttttttt ttaccatggt ccggttaatg 60

t 61

<210> 73
<211> 20
<212> DNA
<213> Artificial Sequence

<220>
<223> Primer

<400> 73
atggccggat ccaagagctc 20

<210> 74
<211> 30
<212> DNA
<213> Artificial Sequence

<220>
<223> Primer

<400> 74
ggccccccct cgaaggatca ggcccttatg 30

## Description

### BACKGROUND OF THE INVENTION

### 1. Field of the Invention

The present invention relates to a translation enhancer for use in a cell-free protein synthesis system and the use thereof. This application is a related application of JP (Tokugan) 2015-046470, which is a Japanese Patent Application filed on March 9, 2015. The present application claims priority right based on the Japanese Application, and cites all of the content disclosed therein.

### 2. Description of the Related Art

A synthesis system that synthesizes a protein without cells is a protein synthesis system that prepares a medium including intracellular elements involved in protein synthesis and conducts from transcription to translation of a template DNA without cells. Various such cell-free protein synthesis systems are known. Such synthesis systems are known as systems that synthesize a protein that is the final product by using template DNA, which is the transcription template, in a medium and systems that synthesize a protein by using mRNA, which is the translation template, in a medium.

In both systems, transcription template DNA must be synthesized as the first raw material. The synthesis of transcription template DNA usually involves first cloning the cDNA that encodes the protein to be synthesized, incorporating the cloned cDNA into a plasmid, and configuring a DNA region for expression including a promoter, coding region, terminator, and the like. Template DNA is then made by cutting the DNA region from the plasmid or by carrying out PCR directly on the plasmid.

The structure of the template DNA is thought to affect the expression efficiency in a cell-free protein synthesis system, and various attempts have been made regarding vectors to configure an expression cassette. For example, the introduction of a specific translation enhancement sequence into the 5' untranslated region has been reported (Patent Documents 1 and 2). Lengthening the life of the mRNA which is the translation template by lengthening the 3' untranslated region and improving the translation efficiency has also been described (Patent Document 3, paragraph 0049). The 3' UTR of mRNA is also reported to preferably to at least 1000 bases (Patent Documents 4 and 5).

The use of a 3' UTR has also been mentioned with regard to a cell-free protein synthesis system using yeast extract (Non-patent Document 1).

### PRIOR ART DOCUMENTS

### PATENT DOCUMENTS

[Patent Document 1] Japanese Laid-open Patent 2009-72207
[Patent Document 2] Japanese Laid-open Patent 2013-158342
[Patent Document 3] Japanese Laid-open Patent 2007-97438
[Patent Document 4] Japanese Laid-open Patent 2008-35701
[Patent Document 5] Japanese Laid-open Patent 2005-247857

### NON-PATENT DOCUMENTS

[Non-patent Document 1] Biotechnology Journal, 2014, 9, 641-651

### SUMMARY OF THE INVENTION

### PROBLEMS TO BE SOLVED BY THE INVENTION

However, transcription template DNA of a corresponding length must be synthesized to add the long 3' UTR recommended in Patent Documents 4, 5, and the like to the mRNA which is the translation template, and mRNA must be synthesized from this template DNA. In any case, the process up to synthesizing the mRNA which is the translation template is not efficient.

Synthesizing a fusion protein with a protein tag added to the C end or N end of a desired protein cloned from a library or the like by providing the template nucleic acid of a cell-free synthesis system with a long 3' UTR was also inconvenient. Specifically, in a nucleic acid amplification reaction using a primer, this is because a primer that synthesizes an amplified fragment having a tag directly under the 3' end of the desired protein and also having a long 3' UTR at the 3' end of the desired protein cannot be designed. Therefore, DNA encoding such a fusion protein was only prepared by chemical synthesis, or DNA encoding the fusion protein was only prepared by ligation.

In Non-patent Document 1, the length of the 3' UTR is said to have no effect in a yeast-derived cell-free protein synthesis system.

This specification provides a translation enhancer that makes it possible to efficiently obtain translation template mRNA in a cell-free protein synthesis system and, in turn, to realize excellent translation efficiency. This specification also provides the use of the said translation enhancer. Furthermore, this specification also provides use focusing on the ability to stabilize mRNA.

### MEANS FOR SOLVING THE PROBLEMS

The present inventors conducted various studies relating to the production of a transcription template of a cell-free protein synthesis system. As a result, they unexpectedly found that transcription template DNA can be obtained by a nucleic acid amplification reaction by a short 3' UTR without using the long 3' UTR required in the past, and that mRNA and protein can be obtained efficiently by using this transcription template DNA in a cell-free protein synthesis system. This specification provides the following means based on these findings.

(1) A translation enhancer to be used for a cell-free protein synthesis system,
   wherein the translation enhancer comprises a nucleic acid of at most 200 bases in length as a 3' untranslated region linked adjacent to the 3' end of a coding region that encodes the amino acid sequence of a desired protein.
(2) The translation enhancer of (1), wherein the 3' untranslated region is at most 100 bases in length.
(3) The translation enhancer of (2), wherein the 3' untranslated region is at most 80 bases in length.
(4) The translation enhancer of (3), wherein the 3' untranslated region is at most 60 bases in length.
(5) The translation enhancer of (4), wherein the 3' untranslated region is at most 40 bases in length.
(6) The translation enhancer of any of (1)-(5), wherein the 3' untranslated region has a poly A sequence of two or more consecutive A.
(7) The translation enhancer of (6), wherein the 3' untranslated region has a poly A sequence of from five to 40 consecutive A.
(8) The translation enhancer of (6) or (7), wherein the 3' untranslated region has a poly A sequence of from five to 20 consecutive A(adenine), and the total base in length is from 10 to 50, preferably from 10 to 40.
(9) The translation enhancer of any of (1)-(8), wherein the 3' untranslated region is a base sequence represented by SEQ ID NO: 6 or 26, a base sequence in which from one to five bases have been substituted, added, inserted, or deleted in the said sequences, or a base sequence having identity of at least 85% with a base sequence represented by SEQ ID NO: 6 or 26, and has a base sequence having 3' untranslated region activity.
(10) The translation enhancer according to any of (1)-(9), wherein the desired protein is a fusion protein provided with a protein tag at the C end of an arbitrary protein.
(11) The translation enhancer according to any of (1)-(10), wherein the desired protein is a fusion protein provided with a protein tag at the N end of an arbitrary protein.
(12) The translation enhancer of any of (1)-(11), which is a translation enhancer for use in a cell-free protein synthesis system including wheat germ extract.
(13) An expression vector to be used for a cell-free protein synthesis system,
   wherein the vector carries a 3' untranslated region of at most 200 bases in length on the 3' side of the insertion site of a coding region that encodes the amino acid sequence of a desired protein.
(14) The vector of (13), wherein the 3' untranslated region carries a region that encodes a protein tag on the 5' side thereof.
(15) A template nucleic acid to be used for a cell-free protein synthesis system,
   wherein the template nucleic acid comprises a promoter region,
   a coding region that encodes the amino acid sequence of a desired protein linked operably by the promoter region,
   and a 3' untranslated region of the coding region, comprising the translation enhancer of any of (1)-(12).
(16) The template nucleic acid of (15), wherein the coding region is a region that encodes a fusion protein provided with a protein tag at the C end of an arbitrary protein as the desired protein.
(17) The template nucleic acid of (15) or (16), wherein the coding region is a region that encodes a fusion protein provided with a protein tag at the N end of an arbitrary protein as the desired protein.
(18) The template nucleic acid of any of (15)-(17), wherein the template nucleic acid is transcription template DNA.
(19) The template nucleic acid of any of (15)-(17), wherein the template nucleic acid is translation template mRNA.
(20) A primer set to be used for DNA polymerase amplification to synthesize transcription template DNA for a cell-free protein synthesis system,
   wherein the primer set comprises
   one or more forward primers,
   one or more reverse primers,
   and the one or more reverse primers are provided with a translation enhancer of any of (1)-(12), or a part thereof.
(21) The primer set of (20), wherein the one or more reverse primers are also provided with a C end protein tag coding region adjacent to the 3' end side of the translation enhancer.
(22) The primer set of (20) or (21), wherein the one or more forward primers have an N end protein tag coding region.
(23) A cell-free protein synthesis kit comprising:
   a primer set of any of (20)-(22),
   and a medium for cell-free protein synthesis.
(24) The kit of (23), wherein the medium for cell-free protein synthesis is derived from wheat germ.
(25) A method for producing transcription template DNA to be used for a cell-free protein synthesis system,
   wherein the method comprises
   a step for synthesizing transcription template DNA by carrying out a nucleic acid amplification reaction on DNA including a coding region of a desired protein using a primer set of any of (20)-(22).
(26) A method for producing a translation template to be used for a cell-free protein synthesis system,
   wherein the method comprises
   a step for synthesizing translation template mRNA using the template nucleic acid of any of (15)-(18) in the absence of cells and in the presence of elements for transcribing transcription template DNA into mRNA.
(27) A method for producing a protein,
   wherein the method comprises
   a step for synthesizing a protein using a template nucleic acid of (19) in the absence of cells and in the presence of elements for translating translation template mRNA into a protein.
(28) An array of translation template DNA,
   wherein the array carries a plurality of template nucleic acids of any of (15)-(18) corresponding to a plurality of proteins as transcription template DNA.
(29) An RNA stabilizer,
   wherein the RNA stabilizer comprises a nucleic acid of at most 200 bases in length as a 3' untranslated region linked adjacent to the 3' end of a coding region that encodes the amino acid sequence of a desired protein.

### BRIEF DESCRIPTION OF THE DRAWINGS

FIG. 1 is a drawing explaining an outline of template nucleic acid in this disclosure by presenting an example thereof;
FIG. 2 is a drawing showing an example of transcription template DNA (for synthesizing a protein having an N end protein tag) primer set of this disclosure;
FIG. 3 is a drawing showing an example of a primer set for obtaining transcription template DNA (for synthesizing a protein having a C end protein tag) of this disclosure;
FIG. 4 is a drawing showing transcription template DNA provided with 3' UTR of various lengths;
FIG. 5 is a drawing showing the results of agarose gel electrophoresis of the first PCR products and second PCR products obtained by the primer sets shown in FIG. 4;
FIG. 6 is a drawing showing the results of agarose gel electrophoresis of translation template mRNA and proteins obtained using the various translation template DNA shown in FIG. 4;
FIG. 7 is a drawing showing an outline of a 3' UTR study;
FIG. 8 is a drawing showing transcription template DNA and primer sets for obtaining a fusion protein having an N end protein tag added to a desired protein;
FIG. 9 is a drawing showing the results of agarose gel electrophoresis of transcription template DNA obtained by the primer sets shown in FIG. 8;
FIG. 10 is a drawing showing the results of agarose gel electrophoresis of translation template mRNA and the results of gel electrophoresis of fusion proteins;
FIG. 11 is a drawing showing transcription template DNA and primer sets for obtaining a fusion protein with a C end protein tag added to a desired protein;
Fig. 12 is a drawing showing the results of agarose gel electrophoresis of the transcription template DNA shown in FIG. 11;
FIG. 13 is a drawing showing the results of gel electrophoresis of translation template mRNA and fusion proteins;
FIG. 14 is a drawing showing transcription template DNA and primer sets for obtaining a fragmentation product by truncation;
FIG. 15 is a drawing showing the results of agarose gel electrophoresis of transcription template DNA obtained by the primer sets shown in FIG. 14;
FIG. 16 is a drawing showing the results of agarose gel electrophoresis of the translation template mRNA and fragmentation products obtained by the various transcription DNA shown in FIG. 14;
FIG. 17 is a drawing showing the results of cell-free protein synthesis using template DNA provided with 3' UTR having 5, 10, 20, 30, and 40 consecutive A;
FIG. 18 is a drawing showing the results of Western blotting of the proteins synthesized.

### DESCRIPTION OF THE PREFERRED EMBODIMENTS

The disclosure of this specification relates to a translation enhancer for use in a cell-free protein synthesis system, template nucleic acids, primer sets, a method for producing transcription template DNA, a method for producing translation template mRNA, a method for producing a protein, a transcription template DNA array, and the like.

The translation enhancer disclosed in this specification functions as a 3' UTR in translation template mRNA even though it is at most 200 bases in length, according to the 3' UTR of the coding region of translation template mRNA (also referred to as a 3' UTR in transcription template DNA) in a cell-free protein synthesis system, and can contribute to efficient translation to a protein.

In addition, since the 3' UTR is constituted of at most 200 bases, the length of the transcription template DNA can be shortened, and transcription template DNA can be obtained efficiently by a nucleic acid amplification reaction.

Furthermore, since the 3' UTR is at most 200 bases in length, this makes it possible to easily obtain transcription template DNA for synthesizing a fusion protein provided with a protein tag at the C end of an arbitrary protein. Specifically, since the 3' UTR is short, a reverse primer provided with part or all of the 3' UTR as a tag region or hybridization region can be designed. Transcription template DNA that encodes the desired protein provided with a protein tag at the C end can be obtained easily by conducted a nucleic acid amplification reaction using this reverse primer.

The template nucleic acids and primer sets used in a cell-free protein synthesis system, methods for producing such transcription template DNA, methods for producing translation template mRNA, methods for producing a protein by a cell-free protein synthesis system, and a transcription template DNA array, which are other embodiments of this disclosure, make it possible to synthesize a desired protein easily and efficiently by using a 3' UTR of such a base length as a translation enhancer, and make it possible to supply the protein for various uses.

Furthermore, the term "cell-free protein synthesis system" in this specification means a translation system (so-called "two-step method") constituted by an element composition that realizes a translation step from at least mRNA derived from a cell to a protein in the absence of cells. A "cell-free protein synthesis system" also means a system (so-called one-step method) provided with both a translation system and a transcription system constituted by an element composition that realizes a transcription step from DNA to mRNA prior to the above translation step in the absence of cells.

Cell-free protein synthesis systems are well known to those skilled in the art. Cell-free protein synthesis systems derived from prokaryotes such as *Escherichia coli* and eukaryotes such as wheat, rabbits, insects, and the like are commercially available to those skilled in the art, and can be prepared appropriately by those skilled in the art. Various synthesis systems such as systems derived from *Escherichia coli,* from wheat germ, from rabbit reticulocytes, and from cultured insect cells can be given as examples of cell-free protein synthesis systems. In addition, a cell-free synthesis system can also construct an artificial synthesis system combining the elements of a transcription system and translation system individually, and is available commercially. The cell-free protein synthesis systems used in the various embodiments of this disclosure are preferably derived from eukaryotes, more preferably derived from wheat germ.

Representative and nonlimiting concrete examples of this disclosure are explained in detail below with reference to the drawings as appropriate. This detailed explanation is intended simply to show those skilled in the art the details for carrying out preferred examples of the present invention; it is not intended to limit the scope of the disclosure. Additional features and inventions also disclosed below can be used separately from or together with other features or inventions to provide a translation enhancer for use in an improved cell-free protein synthesis system, and the use thereof.

In addition, the features and steps disclosed by the following detailed explanation are not essential for carrying out this disclosure in the broadest sense, but are described only to explain representative concrete examples of this disclosure. Furthermore, the various features of the representative concrete examples described above and below and the various features of what is described in the independent and dependent claims do not have to be combined as in the concrete examples described here or in the order listed in providing additional and useful embodiments of this disclosure.

All of the features described in this specification and/or in the claims are intended to be disclosed individually and independently of each other as an initial disclosure of the application and a limitation to the specific items claimed, separate from the constitution of features described in the examples and/or claims. Furthermore, all of the numerical ranges and descriptions relating to groups or populations have the intention of disclosing intermediate configurations thereof as an initial disclosure of the application and a limitation to the specific items claimed.

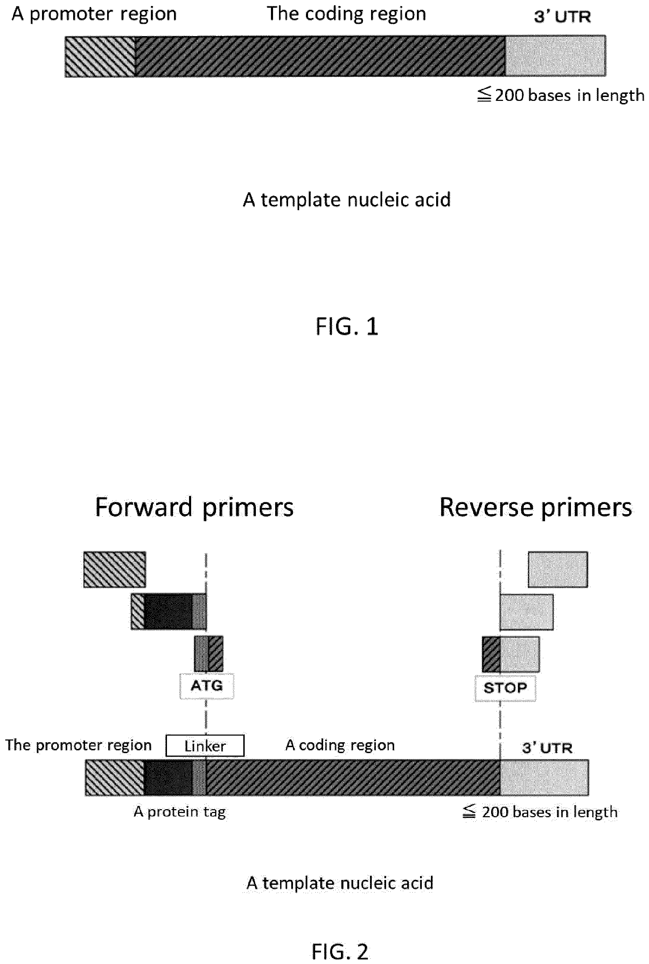

Various forms of this disclosure are explained below with reference to the drawings as appropriate. FIG. 1 shows an example of transcription template DNA and translation template mRNA including the translation enhancer of this disclosure. FIG. 2 is a drawing showing an example of a primer set to be used in a nucleic acid amplification reaction when the desired protein to be synthesized is provided with a protein tag on the C end of an arbitrary protein.

### (Translation enhancer)

The translation enhancer of this disclosure (also referred to as "this enhancer" hereinafter) is an agent to be used in a cell-free protein synthesis system (also referred to simply as "synthesis system" hereinafter). As shown in FIG. 1, this enhancer uses a nucleic acid comprising a predetermined base sequence as a 3' UTR linked adjacent to the 3' end of a coding region that encodes the amino acid sequence of a desired protein in a synthesis system.

For this enhancer, the desired protein to be synthesized is preferably a fusion protein provided with a peptide heterologous to an arbitrary protein, such as a protein tag at the C end of the arbitrary protein. This is because this protein required a very complex procedure associated with cloning and the like to be used in the synthesis system in the past, and high-throughput synthesis was difficult. Similarly, for this enhancer, the desired protein to be synthesized is preferably a fusion protein provided with a heterologous peptide such as a protein tag at the C end of the arbitrary protein.

The heterologous peptide of the protein tag that can be provided at the C end and/or N end of the arbitrary protein is not particularly restricted. Examples of this protein tag include, but are not limited to, a His tag, GST tag, MBP tag, myc tag, FLAG tag, and BCCP tag. In addition, examples of tags that permit visual detection include, but are not limited to, GFP (green fluorescent protein), BFP (blue fluorescent protein), CFP (cyan fluorescent protein), RFP (red fluorescent protein), YFP (yellow fluorescent protein), EGFP (enhanced green fluorescent protein), ECFP (enhanced cyan fluorescent protein), ERFP (enhanced red fluorescent protein), EYFP (enhanced yellow fluorescent protein), TMR (tetramethylrhodamine), luciferase, and the like.

Furthermore, the protein tag is linked directly or via a suitable linker to the N end and/or C end of the arbitrary protein.

This enhancer preferably comprises a nucleic acid no more than 200 bases in length. The base length of this enhancer is far shorter, approximately 1/5-1/15^{th}, in comparison to the base length of conventional 3' UTR. A longer 3' UTR was said to be effective in the past from the viewpoint of improving translation template stability and improving translation efficiency in the synthesis system. The 3' UTR length was generally at least 500 bases in length, typically from 1000 to 3000 bases in length. In contrast to this, this enhancer has a very short base length of no more than 200 bases in length, and its use as a 3' UTR, regardless of its base sequence, makes it possible to synthesize a protein surprisingly efficiently in the synthesis system; also, such a short 3' UTR makes it possible to easily obtain a transcription template and translation template for a fusion protein provided with a protein tag in unprecedented form and, as a result, to easily synthesize the fusion protein.

This enhancer is provided as a DNA double strand on the 3' end side of the DNA when used in the transcription template DNA of the synthesis system. In addition, this enhancer is provided as single-stranded RNA on the 3' end side of the mRNA when used in the translation template mRNA of the synthesis system.

As shown in FIG. 1, the use of a nucleic acid comprising a predetermined base sequence as 3' UTR means use in a state linked directly or indirectly via a suitable intervening sequence to the stop codon of a region that encodes an amino acid sequence in transcription template DNA or translation template mRNA in a cell-free protein synthesis system. When linked indirectly to the stop codon, the intervening sequence is not particularly restricted, but is preferably at most 50 bases in length, more preferably at most 20 bases in length, even more preferably at most 10 bases in length, even more preferably at most 5 bases in length, and even more preferably at most 2 bases in length, in consideration of the length of this enhancer whose length of the 3' UTR is at most 200 bases in length.

This enhancer is preferably shorter than 200 bases in length as long a certain translation efficiency can be maintained. More preferred is at most 150 bases in length, even more preferred is at most 100 bases in length, even more preferred is at most 80 bases in length, and even more preferred is at most 60 bases in length. Even more preferred is at most 40 bases in length. Most preferred is at most 30 bases in length or 20 bases in length.

In addition, the base length of this enhancer is preferably shorter from the viewpoint of primer design when one considers that the transcription template DNA including a region that encodes the desired protein to be synthesized is obtained by a nucleic acid amplification reaction such as PCR. Considering this viewpoint, when the base length of this enhancer is at most 100 bases in length or 80 bases in length, about two or three reverse primers suffice to add this enhancer to the 3' end of the coding region of the transcription template DNA. Furthermore, when the base length of this enhancer is at most 60 bases in length or 40 bases in length, about one or two reverse primers suffice to add it to the 3' end of the coding region. Moreover, when the base length of this enhancer is at most 30 bases in length or 20 bases in length, in the same way, about one reverse primer suffices.

In addition, the base length of this enhancer may be at most 15 bases in length, even at most 10 bases in length.

The lower limit of the base length of this enhancer is not particularly restricted, but at least 5 bases in length is preferred. This is because there is a possibility that the intended coding region will not be translated when the length is less than 5 bases. More preferred is at least 10 bases in length.

A base sequence from the first base of the 5' end of the base sequence (1200 bases) represented by SEQ ID NO: 1 to a base corresponding to a predetermined base length can be given as an example of a preferred embodiment of this enhancer. This enhancer preferably uses a base sequence of from 5 to 400 bases in length, more preferably from 5 to 200 bases in length, from the 5' end of a base sequence represented by SEQ ID NO: 1 as a 3' UTR. More preferred is from 5 to at most 100 bases in length, even more preferred is from 5 to at most 80 bases in length, even more preferred is from 5 to at most 60 bases in length, and even more preferred is from 5 to at most 40 bases in length. 3' UTR of each of these embodiments more preferably comprise only a nucleotide represented by a base sequence of a predetermined base length from the 5' side of the base sequence represented by SEQ ID NO: 1.

This enhancer can also use a nucleic acid having 3' UTR activity that is a base sequence in which from one to several bases have been substituted, added, inserted, or deleted in a base sequence (SEQ ID NO: 6) of 40 bases in length from the 5' end of the base sequence represented by SEQ ID NO: 1. For example, consecutive A on the 5' side are preferably at least 8, but may be at most 20, in the base sequence represented by SEQ ID NO: 1. Preferred is from 10 to 16.

A nucleic acid having 3' UTR activity that is a base sequence having at least 85% identity with the base sequence represented by SEQ ID NO: 6 can also be used. The identity to the base sequence represented by SEQ ID NO: 6 is more preferably at least 90%, even more preferably at least 95%, even more preferably at least 96%, even more preferably at least 97%, even more preferably at least 98%, and even more preferably at least 99%.

The base sequence represented by SEQ ID NO: 6 has a base sequence that can take on a stem-loop structure. Therefore, it is preferable that the base sequence derived from this base sequence also retains this characteristic.

When the base sequence represented by SEQ ID NO: 6 or a nucleic acid provided with a base sequence derived from this base sequence as described above is used as a 3' UTR, the region where the base sequence is provided is preferably at the 5' end or as close as possible to the 5' end of the 3' UTR. As long as a base sequence represented by SEQ ID NO: 6 or a sequence derived therefrom is provided, the base sequence on the 3' end side is not particularly restricted, and various embodiments can be adopted.

This enhancer can have a poly A sequence of at least two consecutive A (adenine). The number of consecutive A in such a poly A sequence is not particularly restricted, but preferred is at least three consecutive A, more preferably at least five consecutive A. Preferred is at least 10 consecutive A, more preferably at least 15, and even more preferably at least 20. The upper limit, for example, is preferably at most 40 consecutive A, more preferably at most 30. Furthermore, for example, the poly A sequence may have from 5 to 30 consecutive A, or from 5 to 20 consecutive A, even from 10 to 30 consecutive A, and even from 10 to 20 consecutive A.

This enhancer may have a plurality of poly A sequences. In this case, the plurality of poly A sequences can be provided in a state in which one or several G, T, and C other than A are interposed. There are preferably at most five interposed bases (nucleotides), more preferably at most three, even more preferably at most two, and even more preferably one. The plurality of adjacent poly A sequences having these interposed bases or sequences can, as a whole, be called a poly A motif.

This enhancer can be provided with a poly A sequence in its polynucleotide, for example, in certain embodiments, at the 5' end or on the 5' end side of this enhancer. In addition, in certain embodiments, a poly A sequence can be provided in the middle of the 5' end and 3' end. Furthermore, in certain embodiments, a poly A sequence can be provided at the 3' end or on the 3' end side. For example, when this enhancer is added as a 3' UTR to the coding region of transcription template DNA, a poly A sequence can be provided immediately after the stop codon, immediately at the 3' end of the stop codon. In this case, the poly A sequence in the polynucleotide of this enhancer is provided on the 5' end side.

When this enhancer has a poly A sequence, it may be constituted from only a poly A sequence or a poly A motif, or may have another sequence comprising arbitrary bases in addition to the poly A sequence or poly A motif. In this case, this enhancer, by including a poly A sequence, can be, as a whole, from 10 to 60 bases in length. Preferably, this enhancer can be from 10 to 50 bases in length. Furthermore, this enhancer can be from 10 to 40 bases in length. For example, this enhancer can have a base sequence of position 17 onward in the base sequence represented by SEQ ID NO: 1. For example, this enhancer may be provided with a consecutive base sequence of from several to at most 100, for example, at most 80, or for example, at most 70, or for example, at most 60, or for example, at most 50, from position 17 of such a base sequence. Having such an additional sequence in addition to the poly A sequence tends to further improve the RNA stabilization and translation enhancing performance.

Such other sequences, for example, preferably have a GC content in the whole (AGTC) of at least 50%, more preferably at least 55%, and even more preferably at least 58%.

As for the suitable number of consecutive A when this enhancer includes a poly A sequence, various primer sets including reverse primers are designed to make it possible to produce a template nucleic acid to provide a poly A sequence of various consecutive A numbers (for example, from about 5 to 50, preferably about 40 at most) as a 3' UTR. Template nucleic acids including poly A sequences of various lengths are produced by these primer sets and used in the cell-free protein synthesis system in which this enhancer is to be used. The suitable poly A length can then be decided based on the amount of protein obtained.

3' UTR activity means that the intended protein can be synthesized when a nucleic acid comprising a predetermined base sequence is used as a 3' UTR in the synthesis system. The method for evaluating the existence and magnitude of 3' UTR activity is not particularly restricted. For example, 3' UTR activity is activity exhibiting a translation level of at least 50%, preferably at least 60%, more preferably at least 70%, even more preferably at least 80%, even more preferably 100%, and even more preferably more than 100%, relative to the translation level by a nucleic acid comprising a base sequence represented by SEQ ID NO: 1, 6, or 26 when a predetermined nucleic acid is used as the 3' UTR under the same conditions except that a nucleic acid comprising a base sequence represented by SEQ ID NO: 1, 6, or 26 is used as the 3' UTR. Furthermore, a synthesis system derived from wheat germ is preferably used as the synthesis system; more preferably a synthesis system derived from wheat germ disclosed in the examples is used.

Identity or similarity in this specification is as known in the art, is decided by comparing sequences, and is a relationship between two or more proteins or two or more polynucleotides. "Identity" in the art means the degree of sequence universality between proteins or polynucleotides as determined by alignment between the protein or polynucleotide sequences or, in some cases, by alignment between a series of such sequences. Similarity means the degree of correlation by alignment between protein or polynucleotide sequences or, in some cases, between a series of partial sequences. More specifically, similarity is decided by the identity and conservancy (substitution to maintain physicochemical properties in specific amino acids or sequences in the sequence). Furthermore, similarity is referred to as similarity in the BLAST homology search results described below. The method for deciding identity and similarity is preferably a method designed for the longest alignment between the sequences compared. Methods for deciding identity and similarity are provided as programs available to the public. For example, a decision can be made using the BLAST (basic local alignment search tool) program of Altschul et al. (for example, Altschul, SF, Gish W, Miller W, Myers EW, Lipman, DJ, J. Mol. Biol., 215: p. 403-410 (1990), Altschul SF, Madden TL, Schaffer AA, Zhang Z, Miller W, Lipman, DJ, Nucleic Acid Res., 25: p. 3398-3402 (1997)). The conditions when using software such as BLAST are not particularly restricted, but it is preferable to use the default values.

This enhancer can be obtained based on chemical or genetic engineering nucleic acid synthesis methods that are themselves known. However, as described below, this enhancer is used as an element of a template nucleic acid or primer, and is commonly obtained as a template nucleic acid or primer.

### (RNA stabilizer)

This enhancer improves the stability of mRNA and, as a result, can improve the translation efficiency to the protein. Specifically, this enhancer can also function as an RNA stabilizer to improve the stability of RNA such as mRNA. Therefore, the base sequences of embodiments in which the base sequence of various embodiments of this enhancer described above has been converted into RNA can serve as a base sequence possessed by RNA as an RNA stabilizer. When mRNA has this sequence on its 3' side, the mRNA is stabilized, translation to the protein can be enhanced, and synthesis of cDNA by reverse transcriptase can also be enhanced. Various other useful types of RNA can also be stabilized.

### (Expression vector)

The expression vector of this disclosure (simply referred to as "this vector" hereinafter) can be provided with this enhancer as part thereof. This enhancer is provided as double-stranded DNA in this vector. This vector can preferably be provided with this enhancer on the 3' side of the insertion site of the coding region that encodes the amino acid sequence of a desired protein. The insertion site will be described below.

This vector can include one or more of this enhancer. When this vector includes a promoter region, this enhancer may be incorporated in the forward direction (5 →3') on the 3' downstream side of the promoter region, or may be incorporated in the reverse direction. The one or more of this enhancer may be the same as or different from each other. In addition, when two or more of this enhancer are provided, their directions may be the same as or different from each other.

This vector can be provided with a region that encodes a protein tag on the 5' side of this enhancer. This makes it possible to obtain the desired protein provided with a protein tag at the C end of an arbitrary protein. In this case, the region that encodes the protein tag can be provided on the 3' end side of the insertion site described below. In addition, this vector may also be provided with a region that encodes a protein tag on the 5' end side of the insertion site. Furthermore, the protein tag is described below.

This vector can also be provided with a promoter region on the 5' side of this enhancer. Examples of the promoter region include, but are not limited to, a conventionally known T7 promoter sequence, SP6 promoter sequence, T3 promoter sequence, and the like.

This vector can be provided with an insertion site of a coding region that encodes the amino acid sequence of a desired protein. General examples of the insertion site include a sequence used as a conventionally known multicloning site, a sequence for homologous recombination, or the like. The insertion site is incorporated on the 5' side of this enhancer.

This vector need not be provided with a poly A sequence and a terminator region on the 3' side of this enhancer. This is because this enhancer itself can enhance translation.

This vector can also be provided with conventionally known drug resistance markers to maintain stability in the host and conventionally known replication origins such as pBR322 Ori, pUC Ori, SV40 Ori, and the like for self-replication in the host.

This vector can be produced using conventionally known gene recombination technology.

Transcription template DNA can be constructed as a template nucleic acid by inserting a region that encodes a desired protein into this vector.

### (Template nucleic acid)

The template nucleic acid of this disclosure (simply referred to as "this template nucleic acid" hereinafter) is one element used in the synthesis system. The template nucleic acid can be transcription template DNA or translation template mRNA. Transcription template DNA may be a circular form such as a plasmid in addition to a linear form synthesized by PCR or the like. Furthermore, in this specification, template nucleic acid means a form of DNA double strand that can be used in a cell-free protein synthesis system. When the template nucleic acid is provided with an enhancer, the antisense strand has a corresponding T sequence when the sense strand, for example, as a poly A sequence as this enhancer. In addition, when transcription template DNA and translation template mRNA are provided with this enhancer, this enhancer is provided on the 3' end side.

This template nucleic acid can be provided with a promoter region, a coding region that encodes the amino acid sequence of a desired protein operably linked by the promoter region, and a 3' UTR comprising the above translation enhancer. According to this template nucleic acid, this template nucleic acid can be obtained efficiently since the 3' UTR is at most 200 bases in length, and, as a result, the desired protein can be synthesized efficiently by the synthesis system. This template nucleic acid is also advantageous in that it does not require elements commonly used on the 3' side of the coding region such as a terminator region, poly A signal, and the like.

A suitable, known promoter region can be used in accordance with the type of synthesis system to be used as the promoter reign in this template nucleic acid.

This template nucleic acid can be provided with a coding region that encodes the amino acid sequence of a desired protein. As was already mentioned, the coding region is preferably provided with a heterologous peptide such as a protein tag on the C end and/or N end of an arbitrary protein as the abovementioned desired protein.

This template nucleic acid can be obtained by known chemical or genetic engineering techniques. However, as described below, it is preferably obtained using a gene or cDNA as a template by utilizing a nucleic acid amplification reaction such as PCR or the like. In addition, translation template mRNA can be obtained by a known translation template mRNA synthesis method used in a two-step method or the like.

### (Primer set)

The primer set of this disclosure (simply referred to as "this primer set" hereinafter) is one element used in the synthesis system. This primer set is used to obtain the transcription template DNA that is this template nucleic acid by a nucleic acid amplification reaction using DNA polymerase.

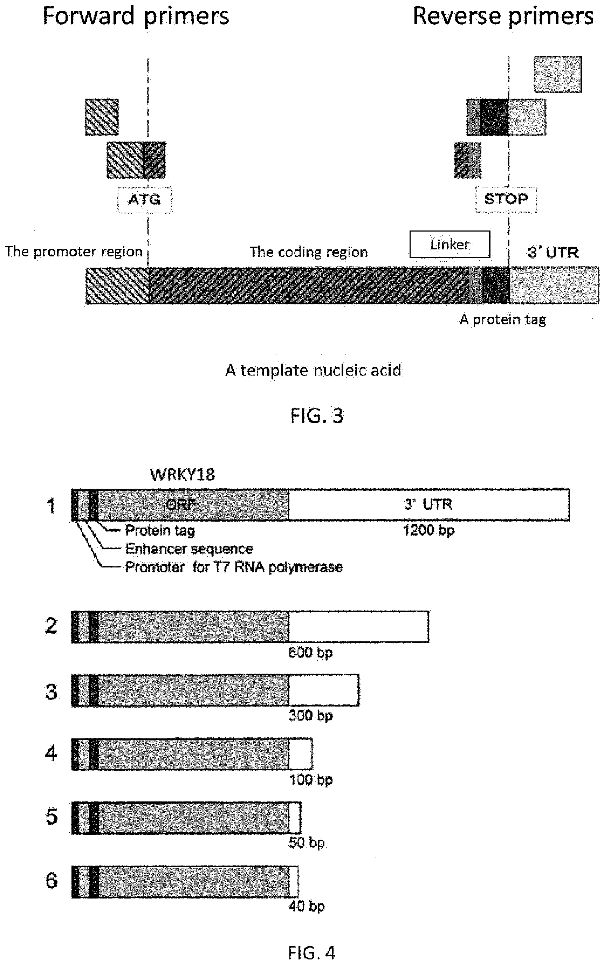

This primer set, as shown in FIGS. 2 and 3, can be provided with one or more forward primers and one or more reverse primers. One or more reverse primers in this primer set can be provided with this enhancer or a part thereof. In other words, this enhancer is provided so that it can be added to the transcription template DNA. This configuration makes it possible to synthesize transcription template DNA provided with this enhancer at the 3' end of the coding region.

Furthermore, the primer sets shown in FIGS. 2 and 3 intend a transcription template nucleic acid for a desired protein provided with a protein tag at the N end of an arbitrary protein and a transcription template nucleic acid for a desired protein provided with a protein tag at the C end of the same, respectively.

The reverse primer provided with this enhancer can take on various embodiments since the configuration adopted and necessary number differ depending on the base length of this enhancer and the configuration of the 3' end of the coding region. For example, as shown in FIG. 2, one embodiment of a reverse primer can be provided with this enhancer or a part thereof on the 5' end side. A reverse primer of this embodiment can also be provided with a hybridization region for hybridizing to the coding region.

In addition, as shown in FIG. 2, in another embodiment of a reverse primer, the whole can adopt an embodiment comprising this enhancer or a part thereof. The embodiment of a reverse primer of this embodiment can be utilized as a universal primer.

Also, for example, as shown in FIG. 3, one embodiment of a reverse primer can be provided with this enhancer or a part thereof on the 5' end side. A reverse primer of this embodiment can also be provided with a hybridization region for hybridizing to the coding region. Also, a reverse primer of this embodiment can also be provided with a coding region of a protein tag on the 3' end side of this enhancer. A reverse primer of this embodiment can be utilized as a universal primer to add a specific protein tag.

In addition, as shown in FIG. 3, in another embodiment of a reverse primer, the whole can adopt an embodiment comprising this enhancer or a part thereof. The embodiment of a reverse primer of this embodiment can be utilized as a universal primer.

Furthermore, reverse primers of various embodiments can be provided suitably with a region that encodes a peptide linker for linking a protein tag or the like to an arbitrary protein.

This primer set can be provided with one or more forward primers. The forward primers, as shown in FIGS. 2 and 3, can also be provided suitably with a hybridization region for hybridizing to the coding region, a linker peptide coding region, and a protein tag coding region.

Furthermore, the reverse primer in this primer set can include this enhancer. In this case, the reverse primer has a base sequence corresponding to the antisense strand of the template nucleic acid. Therefore, for example, when a DNA double-stranded template nucleic acid having a sense strand in which the above poly A sequence is provided in the 3' UTR is provided, the reverse primer will include a sequence complementary to the 3' UTR of the sense strand as this enhancer in part thereof. For example, when this enhancer has a poly A sequence, the reverse primer including this enhancer will include a poly T sequence.

This primer set can generally be obtained by chemical nucleic acid synthesis methods. This primer set explained above can serve as an element of a kit for a synthesis system of this disclosure described below.

### (Kit)

The cell-free protein synthesis kit in this disclosure (simply referred to as "this kit" hereinafter) can be provided with this primer set and a composition for cell-free protein synthesis. Furthermore, this kit can be provided with nucleic acid amplification reagents such as DNA polymerase, various nucleotides, and various reagents such as buffers for carrying out a nucleic acid amplification reaction by PCR using this primer set. Furthermore, this kit can also be provided with solid-phase carriers of various forms such as an array form to hold a plurality of transcription template DNAs synthesized by this primer set. Examples of solid-phase carriers include, but are not limited to, glass well plates and the like.

The composition for cell-free protein synthesis (simply referred to as "this composition" hereinafter) is a composition of elements necessary for protein synthesis derived from various cells or configured artificially based on a two-step method or one-step method. Such compositions are commonly known to those skilled in the art, can be prepared as needed by those skilled in the art, and are also commercially available.

Examples of such compositions include, but are not limited to, conventionally known *Escherichia coli,* wheat, barley, rice, corn, and other such plants of the Gramineae family, and germs of plant seeds such as spinach, extracts and extract liquids extracted from rabbit reticulocytes, and the like. Commercial products of these can be used, and they can also be prepared by methods that are themselves already known, specifically, in the case of *E. coli* extract liquid, according to the method described in Zubay G "Ann. Rev. Genet.," Vol. 7, p. 267-287 (1973), and the like. Examples of commercial cell extract liquids for protein synthesis include, *E. coli* S30 extract for linear templates (manufactured by Promega Inc.) and the like among those derived from *E. coli;* rabbit reticulocyte lysate systems (manufactured by Promega Inc.), and the like among those derived from rabbit reticulocytes; wheat germ extract (manufactured by Promega Inc.), PROTEIOS (manufactured by Toyobo Co., Ltd.), and the like among those derived from wheat germ.

In addition, this composition may include extracts derived from foot animals, extracts derived from mammalian cultured cells, and the like. An extract derived from silkworm tissue is preferably produced by the method described in Japanese Laid-open Patent 2003-235598, and an extract liquid derived from cultured cells is preferably produced by the method described in Japanese Laid-open Patent 2004-215651.

The composition provided in this kit can, for example, be a composition derived from wheat germ.

This kit can be provided with a composition containing one or more of various conventionally known components necessary respectively in a translation system or transcription/translation system, in addition to these extract liquids or extracts. Examples include, but are not limited to, nucleic acid-degrading enzyme inhibitors necessary for protein synthesis, various ions, substrates, phosphotransferase, energy sources, and other such additives for various protein synthesis reactions, as well as RNA polymerase, adenosine triphosphate, guanosine triphosphate, and other such nucleotides, a transcription template or translation template for encoding the protein, and, if desired, a stabilizer containing at least one component selected from inositol, trehalose, mannitol, and the like. Examples of additives for a protein synthesis reaction include amino acids that serve as a substrate, energy sources, potassium salts, magnesium salts, and other various ion sources, buffers, an ATP regeneration system (phosphotransferase), nucleic acid-degrading enzyme inhibitors, tRNA, DTT, and other such reducing agents, polyethylene glycol, 3',5'-cAMP, folates, antimicrobials, and the like. Creatine phosphoric acid and creatine kinase may also be included. Furthermore, for the added concentrations of each of these components, known combination ratios can be decided by one skilled in the art.

The various components necessary to this translation system or transcription/translation system may be prepared separately from the extract or extract liquid, and some or all may be included in the extract or extract liquid in advance.

Furthermore, this kit may be provided with this vector in place of this primer or together with this primer.

### (Method for producing transcription template DNA)

The method for producing transcription template DNA for the cell-free protein synthesis system of this disclosure can be provided with a step for synthesizing transcription template DNA by carrying out a nucleic acid amplification reaction on DNA including a coding region of the desired protein using this primer set. Transcription template DNA is to be obtained as a PCR product by carrying out a nucleic acid amplification reaction using suitable conventionally known PCR amplification reaction conditions on DNA including a coding region that encodes the amino acid sequence of the desired protein using this primer set. Furthermore, when this primer set includes two or more forward primers and/or two or more reverse primers, the intended transcription template DNA is obtained as an amplification product by utilizing two or more amplification reaction conditions (especially temperature cycle and the like) as needed.

Furthermore, transcription template DNA can also be obtained using this vector. Specifically, a template nucleic acid can be obtained by inserting DNA that includes at least a coding region that encodes the amino acid sequence of this protein into this vector. A vector produced in this way can itself be used as transcription template DNA, and a DNA fragment corresponding to the transcription template DNA can also be cut from this vector and used.

The transcription template DNA may be used, for example, in the synthesis system as a PCR reaction solution (specifically, without purifying the transcription template DNA), or may be used in the synthesis system suitably purified, and the like.

### (Method for producing translation template mRNA)

The method for producing a translation template for the cell-free protein synthesis system of this disclosure can be provided with a step for synthesizing translation template mRNA using transcription template DNA in the absence of cells and in the presence of elements for transcribing the transcription template DNA into mRNA. This method, for example, can be carried out in vitro in the presence of at least the various components necessary for the transcription reaction using the composition for cell-free protein synthesis already described. More specifically, translation template mRNA can be obtained by incubating a PCR reaction solution including transcription template DNA or transcription template DNA derived from this vector in the presence of a composition including the components necessary for the transcription reaction such as RNA polymerase compatible with the promoter region provided in the transcription template DNA and a substrate for RNA synthesis (four ribonucleoside triphosphates), for example, for a suitable length of time at approximately 20-60°C, preferably approximately 30-42°C.

This method can be carried out as a synthesis system as part of a transcription/translation system, or can be carried out as a step preceding the application of the translation template mRNA to a translation system. The reaction solution of translation template mRNA obtained in this way can be used in the translation system.

### (Method for producing protein)

The method for producing a protein of this disclosure can be provided with a step for synthesizing a protein using translation template mRNA in the absence of cells and in the presence of elements for translating the translation template mRNA into the protein. This method can also be provided with a step for synthesizing translation template mRNA using transcription template DNA in the absence of cells and in the presence of elements for transcribing the transcription template DNA into mRNA. Furthermore, this method can also be provided with a step for synthesizing the transcription template DNA by carrying out a nucleic acid amplification reaction on DNA that includes a coding region of the desired protein. This method makes it possible to conduct protein synthesis efficiently because translation template mRNA and transcription template DNA including this enhancer are used.

The production of a protein in this method may be realized using a translation system or may be realized using a transcription/translation system.

This method, for example, can be conducted in vitro using the composition for cell-free protein synthesis already described in the presence of at least the various components necessary for a translation reaction. More specifically, the method can be carried out on translation template mRNA by incubating for a suitable length of time at a suitable temperature suited to the translation reaction in the presence of the necessary or appropriate level of amino acids that serve as a substrate, energy sources, various ions, buffers, ATP regeneration system, various degrading enzyme inhibitors, tRNA, reducing agents, polyethylene glycol, 3',5'-cAMP, folates, antimicrobials, and the like.

### (Transcription template array and use thereof)

The array of transcription template DNA of this disclosure can hold a plurality of this template nucleic acid corresponding to a plurality of desired proteins as transcription template DNA. This array makes it possible to synthesize a plurality of desired proteins at once by using them in the synthesis system on an array. This array can be used preferably for various analyses involving proteins, such as protein-protein interaction analysis, protein-DNA interaction analysis, protein post-translation modification analysis, protein structure analysis, synthesis of antigenic proteins, and the like.

Examples of this array include, but are not limited to, 96-well plates that can hold transcription template DNA in each well and plates, disks, and strips on which transcription template DNA is immobilized on a suitable solid-phase carrier, and the like. Furthermore, examples of solid carriers include nonporous materials and porous materials comprising glass materials, ceramic materials, plastic materials, and the like.

### EXAMPLES

The present invention is explained concretely below with reference to examples relating to this disclosure. However, the following examples explain this disclosure and do not limit this disclosure.

### Example 1

### (Protein synthesis evaluation against 3' UTR lengths of 1200-40 bp)

This example is outlined in FIG. 4. In this example, as shown in FIG. 4, PCR was conducted on a plasmid vector that incorporated a T7 promoter, enhancer, protein tag, and WRKY18 coding region, and DNA fragments provided with a 40-1200 bp 3' UTR on the 3' end side of the WRKY18 stop codon were prepared. Furthermore, the base sequences of DNA in which the 3' UTR is 1200 bp, 600 bp, 300 bp, 100 bp, 50 bp, and 40 bp are represented by SEQ ID NO: 1, SEQ ID NO: 2, SEQ ID NO: 3, SEQ ID NO: 4, SEQ ID NO: 5, and SEQ ID NO: 6, respectively.

Proteins were synthesized by a cell-free protein synthesis reaction using the DNA fragments provided with 3' UTR of various lengths. The protocol appears below.

### (1) Primer sequences for obtaining 3' UTR of different lengths

The primer sets for the first PCR and the second PCR of the plasmid vector appear below. Furthermore, each of the individual forward primers shown below were used in the first and second PCR, but the same primer was used for the reverse primer.

**[Table 1]**

| NAME | SEQUENCE | | SEQ.ID. |
|---|---|---|---|
| Forward primer1 | CCAGCAGGGAGGTACTATGGACGGTTCTTCGTTT | | 7 |
| Forward primers2 | CCCGCGAAATTAATACGACTCACTATAG | | 8 |
| | CGACTCACTATAGGGCTCACCTATCTCTCTACACAAAACA TTTCCCTACATACAACTTTCAACTTCCTATTATGGACTACA AGGATGACGATGACAAGCTCCAGCAGGGAGGTACTATG | | 9 |
| Reverse prime1,2 | 1200 bp | CTTTTTGATAATCTCATGACC | 10 |
| | 600 bp | AGTCCTGTCGGGTTTCG | 11 |
| | 300 bp | ATTCATTAATGCAGcTGGC | 12 |
| | 100 bp | AATTAACCCTCACTAAAGG | 13 |
| | 50 bp | AGGATCAGGCCCTTATG | 14 |
| | 40 bp | CCTTATGGCCGGATCC | 15 |

### (2) PCR and protein synthesis

PCR to produce transcription template DNA was conducted under the following conditions using KOD-Plus-Neo (manufactured by Toyobo Co., Ltd.) as the DNA polymerase.

### [Table 2]

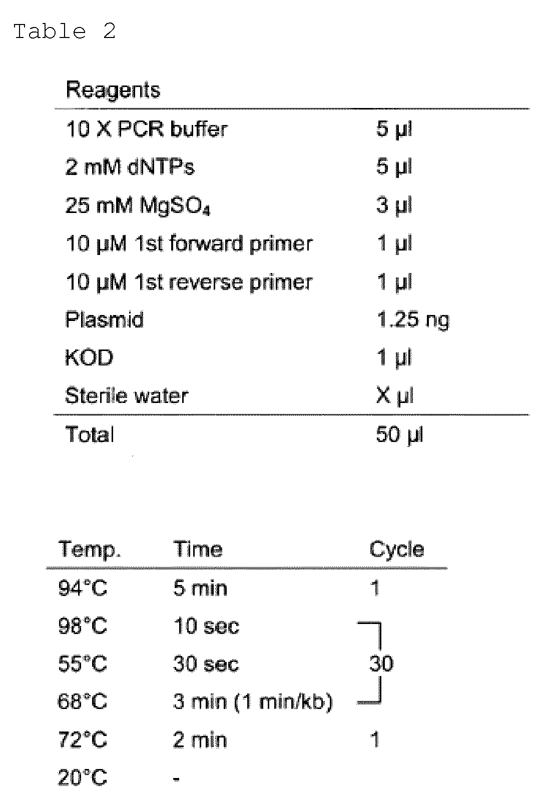

In addition, the second PCR was carried out using the reaction solution composition and reaction conditions shown below in Tables 3 and 4. In addition, 1 µL of the first PCR product was used.

**[Table 3]**

| Reagents | |
|---|---|
| 10 X PCR buffer | 5 µl |
| 2 mM dNTPs | 5 µl |
| 25 mM MgSO₄ | 3 µl |
| 10 µM SpT7u primer | 1 µl |
| 100 nM SpT7dFLAG/His/HA/BAP | 1 µl |
| 10 µM Reverse_U | 1 µl |
| 100 nM deReverse | 1 µl |
| 1st PCR product | 1-5 µl* |
| KOD | 1 µl |
| Sterile water | X µl |
| Total | 50 µl |

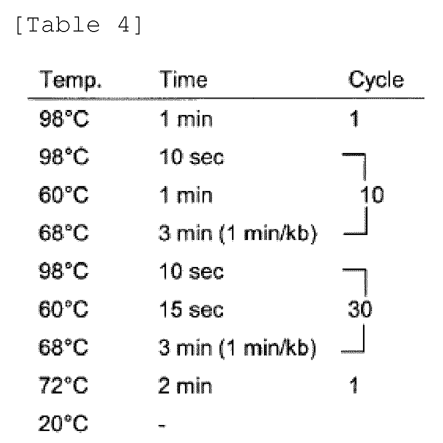

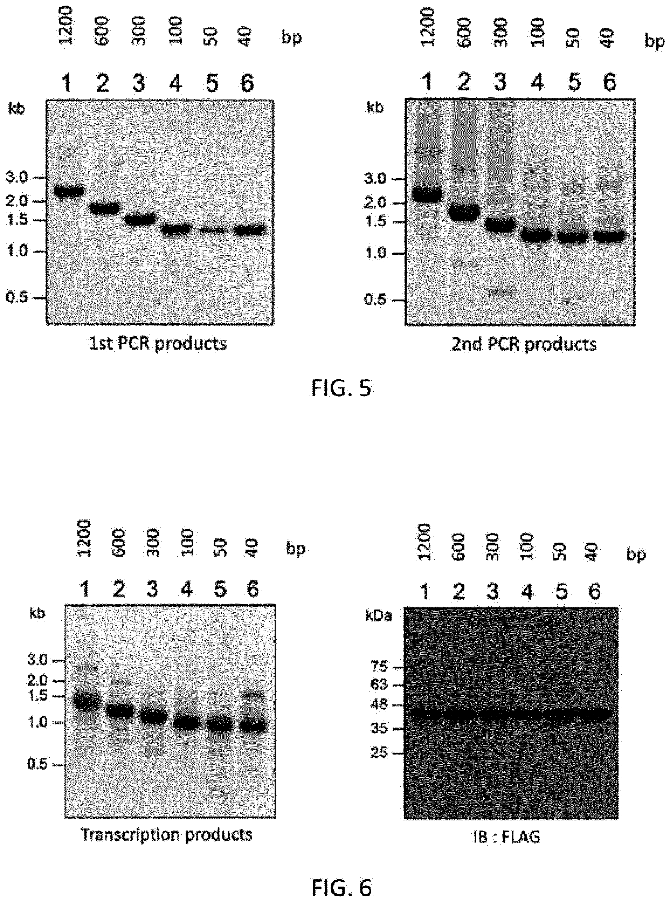

FIG. 5 shows the results of conducting agarose gel electrophoresis of the first and second PCR products by the usual method. As shown in FIG. 5, the intended transcription template DNA was understood to have been obtained as the first and second PCR products.

### (Transcription reaction)

Next, translation template mRNA was produced using the transcription template DNA produced. The transcription reaction was conducted for three hours at 37°C using the following reaction solution of a MEGAscript T7 Transcription Kit (Invitrogen) and 2.5 µL of the second PCR reaction solution (containing transcription template DNA) previously produced.

**[Table 5]**

| Reagents (one reaction) | |
|---|---|
| 10 X Transcription buffer (TB) | 2.5 µl |
| 25 mM NTPs | 2.5 µl |
| RNase Inhibitor (RI) | 0.06 µl |
| 0,1 M DTT | 1.25 µl |
| 1 X T7 RNA Polymerase | 1 µl |
| 2nd PCR product | 2.5 µl |
| RNase-free water (DEPC) | 15.19 µl |
| Total | 25 µl |

After the transcription reaction had been completed, 1 µL of transcription product was confirmed using agarose gel electrophoresis. The results are shown on the left side of FIG. 6. After confirmation, 10 µL of 4 M ammonium acetate was added to 25 µL of the transcription reaction solution and mixed thoroughly, and 100 µL of 100% ethanol was also added and mixed by inverting. After centrifuging for several seconds by a desktop centrifuge, the mixture was allowed to stand for 20 minutes on ice, then centrifuged (15,000 rpm, 20 min, 4°C). After removing the supernatant, centrifugation was conducted for several seconds by desktop centrifuge. The supernatant was again removed, and the precipitate was allowed to stand until dry. Thereafter, 70 µL of RNase-free water (DEPC water) was added to 25 µL of the transcription reaction solution, and the precipitate was suspended thoroughly by a chip. This suspension was taken as the translation template mRNA solution.

### (Translation reaction)

Next, reaction was conducted for approximately 10 hours by incubating at 16°C using a translation reaction solution of the following composition. Furthermore, a composition solution excluding the translation template mRNA from the following composition was prepared, the translation template mRNA was added after the composition solution had returned to room temperature thereafter, and the reaction was conducted by pumping so as not to create foam.

**[Table 6]**

| Reagents | |
|---|---|
| Wheat germ extract | 20 µl |
| 4 X Amino acid mix | 20 µl |
| mRNA | 70 µl |
| Total | 110 µl |

After the reaction, the reaction solution was recovered in an Eppendorf tube and centrifuged (15,000 rpm, 10 min, 4°C), and the supernatant was stored at -80°C. The results of Western blotting of the proteins obtained are shown in the right side of FIG. 6.

As shown in FIG. 6, transcription template DNA was synthesized in accordance with 3' UTR of different lengths, and the intended protein could be obtained from each of the transcription template DNAs. It was understood from these results that a protein can be synthesized regardless of the 3' UTR length, even by transcription template DNA having a base length of 40 as the 3' UTR.

### Example 2

### (Evaluation of three kinds of 3' UTR)

In this example, the translation efficiency by the 3' UTR of two vectors, pENTR/D-TOPO and pDONR221, and the 3' UTR used in Example 1 was evaluated.

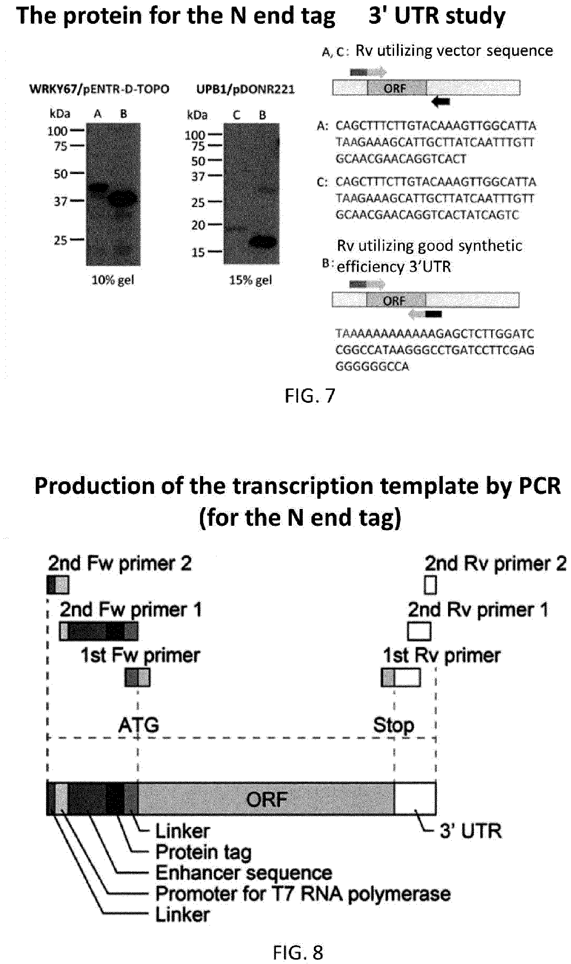

As shown in FIG. 7, first and second PCR were conducted in the same way as in Example 1 using the following primer sets on pENTR/D-TOPO that cloned the coding region of WRKY67 and pDONR221 that cloned the coding region of UPB1, and transcription template DNA was prepared. Specifically, as shown in FIG. 7, transcription template DNA with the 3' UTR derived from the vector using WRKY67/pENTR/D-TOPO (sequence shown as A in FIG. 7 (however, sequence from after the stop codon)) added and transcription template DNA with the 3' UTR used in Example 1 (sequence shown as B in FIG. 7 (however, sequence from after the stop codon) (AAAAAAAAAAGAGCTCTTGGATCCGGCCATAAGGGCC) (SEQ ID NO: 26)) added were prepared. Transcription template DNA with the 3' UTR derived from the vector using UPB1/pDONR221 (sequence shown as C in FIG. 7 (however, sequence from after the stop codon)) added and transcription template DNA with the 3' UTR used in Example 1 (part of sequence shown as B in FIG. 7) added were also prepared.

**[Table 7]**

| NAME | | | SEQUENCE | SEQ.ID. |
|---|---|---|---|---|
| 1st | Forward (WRKY67) | | CCAGCAGGGAGGTACTATGGTTTCCAACAT TGAT | 16 |
| | Forward (UPB1) | | CCAGCAGGGAGGTACTATGGGTGTAACATT AGAA | 17 |
| | A | Reverse | GGGATATCAGCTGGATGGCAA | 18 |
| | B | Reverse (WRKY67) | CCTTATGGCCGGATCCAAGAGCTCTTTTTT TTTTTTAATCAAAAGCAGAAATGTT | 19 |
| | | Reverse (UPB1) | CCTTATGGCCGGATCCAAGAGCTCTTTTTT TTTTTTAAACACAGTTAGTTTCGGT | 20 |
| | C | Rreverse | GGGATATCAGCTGGATGGC | 21 |
| 2nd | SpT7u | | **CCCGCGAAATTAATACGACTCACTATAG** | 8 |
| | SpT7dFLAG | | CGACTCACTATAGGGCTCACCTATCTCT CTACACAAAACATTTCCCTACATACAAC TTTCAACTTCCTATTATGGACTACAAGGA TGACGATGACAAG CTCCAGCAGGGA GGTACTATG | 9 |
| | A | Reverse | **AGTGA**CCTGTTCGTTGC**AAC** | 22 |
| | B | Reverse_U | GGCCCCCCCTCGAAGG | 23 |
| | | deReverse | CCCTCGAAGGATCAGGCCCTTATGGCCGGA TCCAA | 24 |
| | C | Reverse | GACTGATAGTGACCTGTTCG | 25 |

Translation template mRNA was prepared from the transcription template DNA obtained, again in the same way as in Example 1, and proteins were synthesized. The results of Western blotting of the proteins synthesized are also shown in FIG. 7.

As shown in FIG. 7, both 3' UTR could synthesize protein, but the 3' UTR including the poly A sequence of ten consecutive A identified by the base sequence shown as B was understood to be advantageous for high expression.

### Example 3

### (N end tag addition by a 3' UTR approximately 40 bases in length)

In this example, proteins having a FLAG tag added to the N end of each of six proteins (MKK1, MKK2, MKK4, MKK5, MKK6, MKK9) using the 3' UTR 37 bases in length (SEQ ID NO: 26) evaluated in Example 2 as a translation enhancer were synthesized by preparing transcription template DNA based on the primer design shown in FIG. 8.

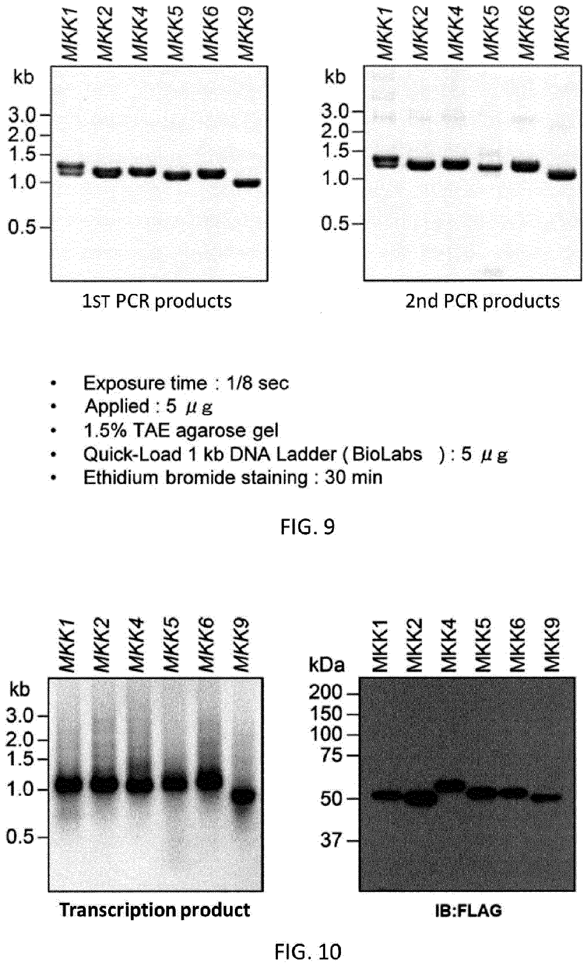

First and second PCR were conducted in the same way as in Example 1 on a vector pDONR221 that cloned the cDNA that encodes each of these proteins using the following primer sets, and transcription template DNA was prepared. FIG. 9 shows the results of agarose gel electrophoresis of the first and second PCR products. Furthermore, translation template mRNA was prepared in the same way as in Example 1, and proteins were finally obtained by a cell-free protein synthesis system. FIG. 10 shows the results of agarose gel electrophoresis of the translation template mRNA and the results of Western blotting of the proteins synthesized.

**[Table 8]**

| | NAME | | SEQUENCE | SEQ.ID. |
|---|---|---|---|---|
| 1st | Forward primer | MKK1 | CCAGCAGGGAGGTACTATGAACAGAGGAAG CTTA | 27 |
| | | MKK2 | CCAGCAGGGAGGTACTATGAAGAAAGGTGG ATTC | 28 |
| | | MKK4 | CCAGCAGGGAGGTACTATGAGACCGATTCA ATCG | 29 |
| | | MKK5 | CCAGCAGGGAGGTACTATGAAACCGATTCA ATCT | 30 |
| | | MKK6 | CCAGCAGGGAGGTACTATGGTGAAGATCAA ATCG | 31 |
| | | MKK9 | CCAGCAGGGAGGTACTATGGCTTTAGTACGT GAA | 32 |
| | Reverse primer | MKK1 | CCTTATGGCCGGATCCAAGAGCTCTTTTTTTT TTTTTAGTTAGCAAGTGGGGGAAT | 33 |
| | | MKK2 | CCTTATGGCCGGATCCAAGAGCTCTTTTTTTT TTTTTACACGGAGAACGTACCAGA | 34 |
| | | MKK4 | CCTTATGGCCGGATCCAAGAGCTCTTTTTTT TTTTTATGTGGTTGGAGAAGAAGA | 35 |
| | | MKK5 | CCTTATGGCCGGATCCAAGAGCTCTTTTTTTT TTTTTAAGAGGCAGAAGGAAGAGG | 36 |
| | | MKK6 | CCTTATGGCCGGATCCAAGAGCTCTTTTTTT TTTTTATCTAAGGTAGTTAACAGG | 37 |
| | | MKK9 | CCTTATGGCCGGATCCAAGAGCTCTTTTTTT TTTTTTAAAGATCTTCCCGGAGAAA | 38 |
| 2nd | Forward primer | SpT7u | **CCCGCGAAATTAATACGACTCACTATAG** | 8 |
| | | FLAG-tag | CGACTCACTATAGGGCTCACCTATCTCT CTACACAAAACATTTCCCTACATACAACT TTCAACTTCCTATTATGGACTACAAGGAT GACGATGACAAG CTCCAGCAGGGAG GTACTATG | 9 |
| | Reverse primer | Reverse_U | GGCCCCCCCTCGAAGG | 23 |
| | | deReverse | CCCTCGAAGGATCAGGCCCCTTATGGC CGGATCCAA | 24 |

As shown in FIG. 9, the first and second PCR products both had the intended lengths, and it was understood that transcription template DNA that encodes each protein could be obtained. As shown in FIG. 10 it was understood that translation template mRNA corresponding to the transcription template DNA and the intended proteins could be obtained.

Based on the above, it was understood that fusion proteins could be obtained by an embodiment that provided a FLAG tag to the N end of each protein even by using a 3' UTR approximately 40 bases in length.

### Example 4

### (C end tag addition by a 3' UTR approximately 40 bases in length)

In this example, proteins having a FLAG tag added to the C end of each of six proteins (ERF1, WRKY18, TGA2, NPR1, MYC2, phyB) using the 3' UTR 37 bases in length (SEQ ID NO: 26) evaluated in Example 2 as a translation enhancer were synthesized by preparing transcription template DNA based on the primer design shown in FIG, 11. Furthermore, the reaction solution of the second PCR for preparing transcription template DNA was as follows.

**[Table 9]**

| Reagents | |
|---|---|
| 10 X PCR buffer | 5 µl |
| 2 mM dNTPs | 5 µl |
| 25 mM MgSO₄ | 3 µl |
| 10 µM T7Eu primer | 1 µl |
| 100 nM FLAG/His/HA/BAP-deReverse | 1 µl |
| 10 µM Reverse_U | 1 µl |
| 1st PCR product | 1-5 µl* |
| KOD | 1 µl |
| Sterile water | X µl |
| Total | 50 µl |

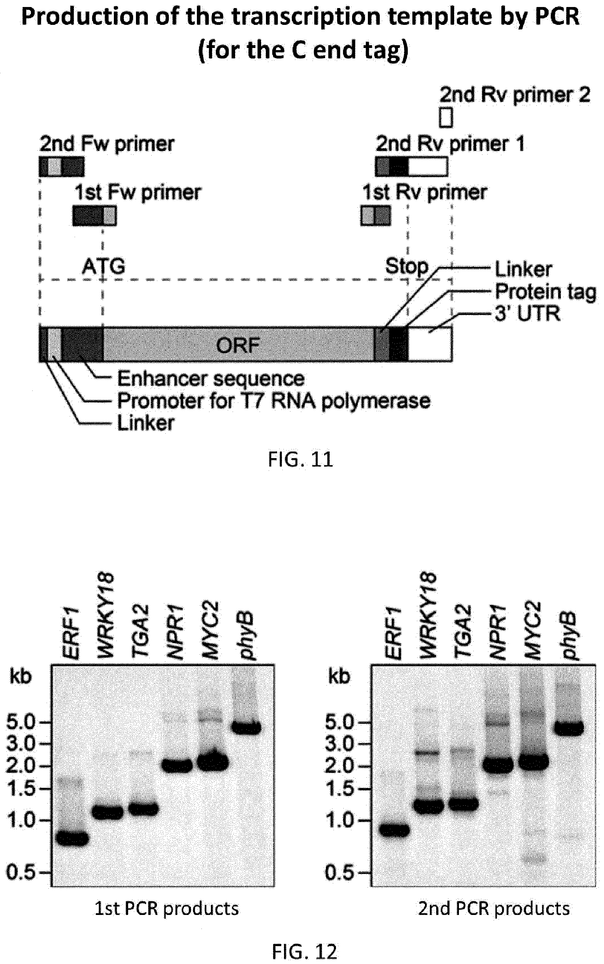

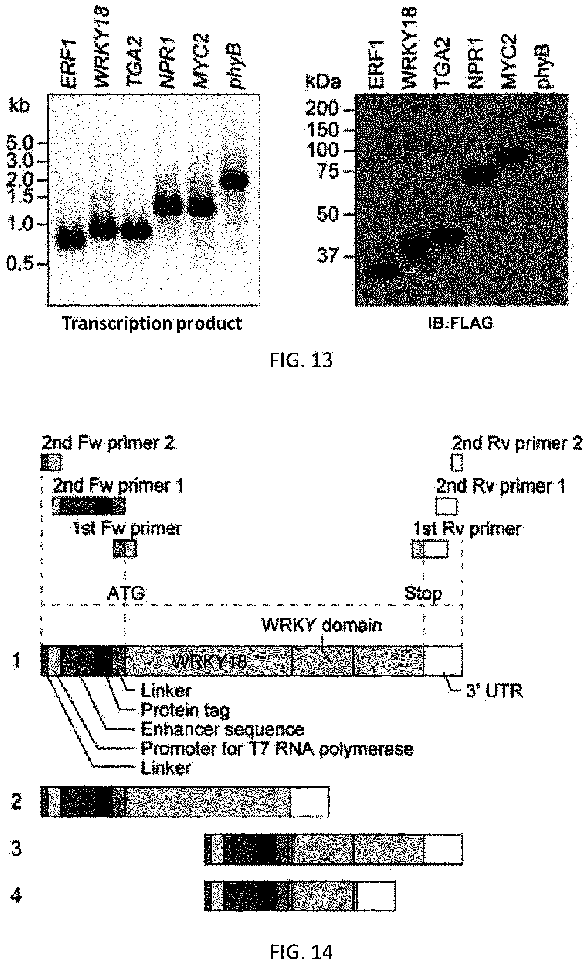

First and second PCR were conducted in the same way as in Example 1 on a vector pDONR221 that cloned the cDNA that encodes each of these proteins using the following primer sets, and transcription template DNA was prepared. FIG. 12 shows the results of agarose gel electrophoresis of the first and second PCR products. Furthermore, translation template mRNA was prepared in the same way as in Example 1, and proteins were finally obtained by a cell-free protein synthesis system. FIG. 13 shows the results of agarose gel electrophoresis of the translation template mRNA and the results of Western blotting of the proteins synthesized.

**[Table 10]**

| | NAME | SEQUENCE | | SEQ.ID. |
|---|---|---|---|---|
| 1st | Forward primer | ERF1 | CACAAAACATTTCCCTACATACAACTTTCAA CTTCCTATTATGGATCCATTTTTAATT | 39 |
| | | WRKY18 | CACAAAACATTTCCCTACATACAACTTTCAA CTTCCTATTATGGACGGTTCTTCGTTT | 40 |
| | | TGA2 | CACAAAACATTTCCCTACATACAACTTTCAA CTTCCTATTATGGCTGATACCAGTCCG | 41 |
| | | NPR1 | CACAAAACATTTCCCTACATACAACTTTCAA CTTCCTATTATGGACACCACCATTGAT | 42 |
| | | MYC2 | CACAAAACATTTCCCTACATACAACTTTCAA CTTCCTATTATGACTGATTACCGGCTA | 43 |
| | | phyB | CACAAAACATTTCCCTACATACAACTTTCAA CTTCCTATTATGGTTTCCGGAGTCGGG | 44 |
| | Reverse primer | ERF1 | AGTACCTCCCTGCTGGAGACCCCAAGTCCC ACTATTTTC | 45 |
| | | WRKY18 | AGTACCTCCCTGCTGGAGACCTGTTCTAGAT TGCTCCAT | 46 |
| | | TGA2 | AGTACCTCCCTGCTGGAGACCCTCTCTGGG TCGAGCAAG | 47 |
| | | NPR1 | AGTACCTCCCTGCTGGAGACCCCGACGACG ATGAGAGAG | 48 |
| | | MYC2 | AGTACCTCCCTGCTGGAGACCACCGATTTTT GAAATCAA | 49 |
| | | phyB | AGTACCTCCCTGCTGGAGACCATATGGCATC ATCAGCAT | 50 |
| 2nd | Forward primer | T7Eu-primer | CCCGCGAAATTAATACGACTCACTATAG GGCTCACCTATCTCTCTACACAAAACATT TCC | 51 |
| | Reverse primer | Reverse_U | GGCCCCCCCTCGAAGG | 23 |
| | | FLAG-deReverse | CCCTCGAAGGATCAGGCCCTTATGGC CGGATCCAAGAGCTCTTTTTTTTTTTTACTTG TCATCGTCATCCTTGTAGTC AGTACCTCC CTGCTGG | 52 |

As shown in FIG. 12, the first and second PCR products both had the intended lengths, and it was understood that transcription template DNA that encodes each protein could be obtained. As shown in FIG. 13, it was understood that translation template mRNA corresponding to the transcription template DNA and the intended proteins could be obtained.

Based on the above, it was understood that fusion proteins could be obtained by an embodiment that provided a FLAG tag to the C end of each protein even by using a 3' UTR approximately 40 bases in length.

### Example 5

### (Example of truncation)

In this example, a protein (WRKY18) was truncated as shown in FIG. 14 using the 3' UTR 37 bases in length (SEQ ID NO: 26) evaluated in Example 2 as a translation enhancer, and fusion proteins 1-4 having a FLAG tag added to the N end of each fragment were synthesized.

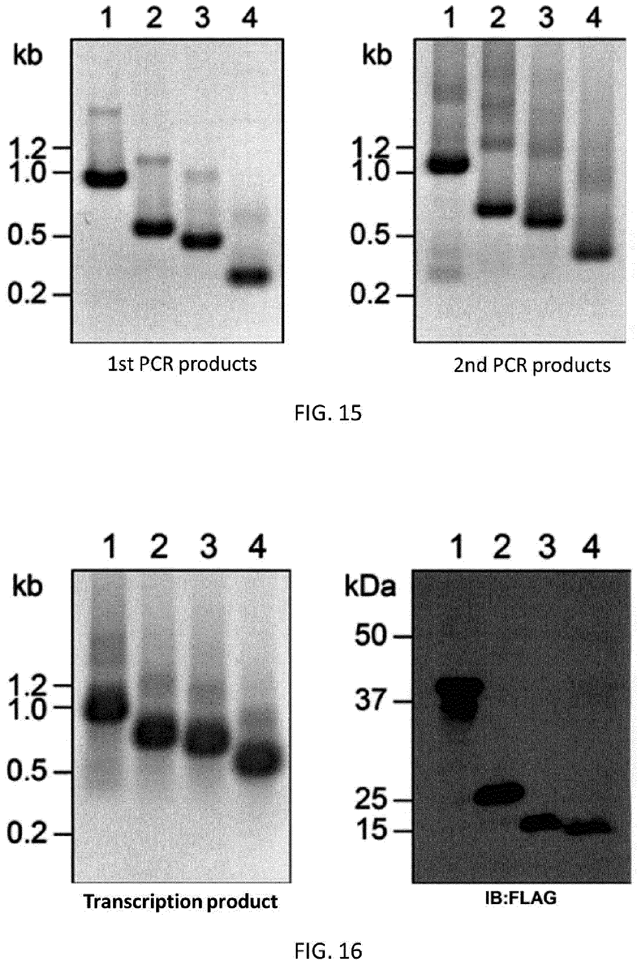

First and second PCR were conducted in the same way as in Example 1 on a vector pDONR221 that cloned the cDNA that encodes these proteins using the following primer sets, and transcription template DNA was prepared. FIG. 15 shows the results of agarose gel electrophoresis of the first and second PCR products. Furthermore, translation template mRNA was prepared in the same way as in Example 1, and proteins were finally obtained by a cell-free protein synthesis system. FIG. 16 shows the results of agarose gel electrophoresis of the translation template mRNA and the results of Western blotting of the proteins synthesized.

**[Table 11]**

| | NAME | SEQUENCE | | SEQ.ID. |
|---|---|---|---|---|
| 1st | Forward primer | 1 | CCAGCAGGGAGGTACTATGGACGGTTCTTC GTTT | 53 |
| | | 2 | CCAGCAGGGAGGTACTATGGACGGTTCTTC GTTT | 54 |
| | | 3 | CCAGCAGGGAGGTACTACTGAAACATCGGA CACA | 55 |
| | | 4 | CCAGCAGGGAGGTACTATGAATGCTTCTGAA GGG | 56 |
| | Reverse primer | I | CCTTATGGCCGGATCCAAGAGCTCTTTTTTT TTTTTATGTTCTAGATTGCTCCAT | 57 |
| | | 2 | CCTTATGGCCGGATCCAAGAGCTCTTTTTTTT TTTTTACAAGCTTGTGTCCGA | 58 |
| | | 3 | CCTTATGGCCGGATCCAAGAGCTCTTTTTTT TTTTTATGTAGCATCCCCTTC | 59 |
| | | 4 | CCTTATGGCCGGATCCAAGAGCTCTTTTTTTT TTTTTTATGTTCTAGATTGCTCCAT | 60 |
| 2nd | SpT7u | | **CCCGCGAAATTAATACGACTCACTATAG** | 8 |
| | SpT7dFLAG | | CGACTCACTATAGGGCTCACCTATCTCT CTACACAAAACATTTCCCTACATACAACT TTCAACTTCCTATTATGGACTACAAGGAT GACGATGACAAG CTCCAGCAGGGAG GTACTATG | 9 |
| | Reverse primer | Reverse_U | GGCCCCCCCTCGAAGG | 23 |
| | | deReverse | CCCTCGAAGGATCAGGCCCTTATGGCCGGA TCCAA | 24 |

As shown in FIG. 15, the first and second PCR products both had the intended lengths, and it was understood that transcription template DNA that encodes each protein could be obtained. As shown in FIG. 16, it was understood that translation template mRNA corresponding to the transcription template DNA and the intended proteins could be obtained.

Based on the above, it was understood that fusion proteins could be obtained as a truncated fragment of each type by an embodiment that provided a FLAG tag to the N end of each protein even by using a 3' UTR approximately 40 bases in length.

### Example 6

### (Evaluation of enhancer base length)

In this example, the sequence and base length that can be used as a 3' UTR were evaluated. Poly A sequences of different numbers of consecutive A were added as a 3' UTR after the stop codon of a base sequence that encodes Arabidopsis transcriptional cofactor NPR1 and transcription factor AREB2 using the following primers, and the amount of each protein synthesized was evaluated.

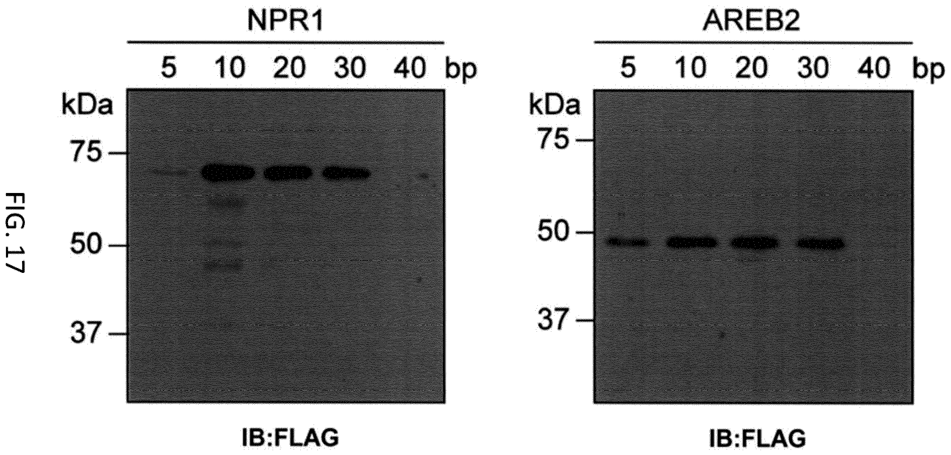

Specifically, primer sets including a reverse primer for obtaining a template nucleic acid having a poly A sequence of 5, 10, 20, 30, and 40 consecutive A added immediately after the stop codon of the base sequence that encodes each protein were synthesized. Furthermore, forward primers were designed to be able to add a FLAG protein to the N end of each protein. First and second PCR were conducted on a vector pDONR221 cloned to include the coding region of these transcription factors and the like in the same way as in Example 1 using these primer sets, transcription template DNA and mRNA were prepared, and proteins were finally obtained by a cell-free protein synthesis system. FIG. 17 shows the results of Western blotting of the proteins synthesized.

**[Table 12]**

| NAME | | | SEQUENCE | SEQ.ID. |
|---|---|---|---|---|
| 1st PCR primer | Forward | NPR1 | CCAGCAGGGAGGTACTATGGACACCACCATTGAT | 61 |
| | | AREB2 | CCAGCAGGGAGGTACTATGGGAACTCACATCAAT | 62 |
| | Reverse | NPR1-A5 | TTTTTTTACCGACGACGATGAGAGAG | 63 |
| | | NPR1-A10 | TTTTTTTTTTTTACCGACGACGATGAGAGAG | 64 |
| | | NPR1-A20 | TTTTTTTTTTTTTTTTTTTTTTACCGACGACGATGAGAGAG | 65 |
| | | NPR1-A30 | TTTTTTTTTTTTTTTTTTTTTTTTTTTTTTTACCGACGACGATGAGAGAG | 66 |
| | | NPR1-A40 | TTTTTTTTTTTTTTTTTTTTTTTTTTTTTTTTTTTTTTTTTTACCGACGACGATGAGAGAG | 67 |
| | | AREB2-A5 | TTTTTTTACGATGGTCCGGTTAATGT | 68 |
| | | AREB2-A10 | TTTTTTTTTTTTACCATGGTCCGGTTAATGT | 69 |
| | | AREB2-A20 | TTTTTTTTTTTTTTTTTTTTTTACCATGGTCGGGTTAATGT | 70 |
| | | AREB2-A30 | TTTTTTTTTTTTTTTTTTTTTTTTTTTTTTTTACCATGGTCCGGTTAATGT | 71 |
| | | AREB2-A40 | TTTTTTTTTTTTTTTTTTTTTTTTTTTTTTTTTTTTTTTTTTACCATGGTCCGGTTAATGT | 72 |
| 2nd PCR primer | Forward | SpT7u | CCGGCGAAATTAATACGACTGACTATAG | 8 |
| | | SpT7dFLAG | CGACTCACTATAGGGCTCACCTATCTCTCTACACAAAACATTTGCCTACATACAAGTTTC AACTTCCTATTATGGACTACAAGGATGACGATGACAAGCTGCAGCAGGGAGGTACTATG | 9 |
| | Reverse | NPR1-A5 | 1st primer Used same primer as the first primer | 63 |
| | | NPR1-A10 | | 64 |
| | | NPR1-A20 | | 65 |
| | | NPR1-A30 | | 66 |
| | | NPR1-A40 | | 67 |
| | | AREB2-A5 | | 68 |
| | | AREB2-A10 | | 69 |
| | | AREB2-A20 | | 70 |
| | | AREB2-A30 | | 71 |
| | | AREB2-A40 | | 72 |

As shown in FIG. 17, adequate protein synthesis could not be assured by a poly A sequence of five consecutive A as the 3' UTR alone for NPR1 protein, but adequate protein synthesis could be assured by a poly A of from 10 to 30 consecutive A. Although not particularly restricted, the maximum amount was synthesized by a poly A of ten consecutive A.

In addition, fairly adequate protein synthesis could be assured by a poly A sequence of from 5 to 40 consecutive A as the 3' UTR for the AREB2 protein. Good protein synthesis could be assured by a poly A sequence of 10-20 consecutive A.

Based on the above, it was understood that the mRNA is stabilized and protein can be synthesized by a cell-free protein synthesis system even by a poly A sequence of at least five consecutive A as the 3' UTR. In addition, it was understood that the number of consecutive A in the poly A can be selected as is appropriate within a range of from about five to 40, as needed.

### Example 7

### (Evaluation of enhancer base sequence)

In this example, the base sequence that can be used as a 3' UTR was evaluated. A poly A sequence of 10 consecutive A, a sequence comprising a total of 20 bases provided with a poly A sequence of 10 consecutive A and a base sequence from positions 17 to 26 following a poly A of a base sequence represented by SEQ ID NO: 1, and a sequence comprising a total of 47 bases provided with a poly A sequence of 10 consecutive A and a base sequence from positions 17 to 63 following a poly A of a base sequence represented by SEQ ID NO: 1 were added as a 3' UTR after the stop codon of a base sequence that encodes Arabidopsis transcription cofactor NPR1 using the following primers, and the amount of each protein synthesized was evaluated.

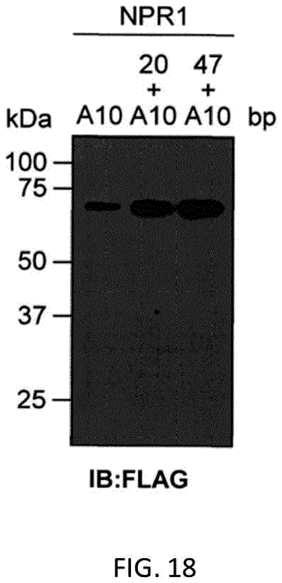

Specifically, primer sets including a reverse primer for obtaining a template nucleic acid having a poly A sequence of 10 consecutive A directly following the stop codon of a base sequence that encodes each protein and also having a base sequence comprising bases of from position 17 to a predetermined number within in the above base sequence represented by SEQ ID NO: 1 adjacent to the poly A sequence of 10 consecutive A, were synthesized. Furthermore, forward primers were designed to be able to add a FLAG protein to the N end of each protein. First and second PCR were conducted on a vector pDONR221 cloned to include the coding region of these transcription factors in the same way as in Example 1 using these primer sets, transcription template DNA and mRNA were prepared, and proteins were finally obtained by a cell-free protein synthesis system. FIG. 18 shows the results of Western blotting of the proteins synthesized.

**[Table 13]**

| NAME | | | SEQUENCE | SEQ.ID. |
|---|---|---|---|---|
| 1st PCR primer | Forward NPR1 | | CCAGCAGGGAGGTACTATGGACACCACCATTGAT | 61 |
| | Reverse | NPR1-A10 | TTTTTTTTTTTTACCGACGACGATGAGAGAG | 64 |
| | | NPR1-A10+20 | ATGGCCGGATCCAAGAGCTCTTTTTTTTTTTTACCGACGACGATGAGAG | 65 |
| | | NPR1-A10+47 | CCTTATGGCCGGATCCAAGAGCTCTTTTTTTTTTTTACCGACGACGATGAGAGAG | 72 |
| 2nd PCR primer | Forward | SpT7u | CCCGCGAAATTAATACGACTCACTATAG | 8 |
| | | SpT7dFLAG | CGACTCACTATAGGGCTCACCTATCTCTCTACACAAAACATTTCCCTACATACAACTTTCA ACTTCCTATTATGGACTACAAGGATGACGATGACAAGCTCCAGCAGGGAGGTACTATG | 9 |
| | Reverse | NPR1-A10 | TTTTTTTTTTTTACCGACGACGATGAGAGAG | 64 |
| | | NPR1-A10+20 | ATGGCCGGATCCAAGAGCTC | 73 |
| | | NPR1-A10+47 | GGCCCCCCCTCGAAGGATCAGGCCCTTATG | 74 |

As shown in FIG. 18, it was understood that adding a polynucleotide of a certain length to a poly A sequence of 10 consecutive A improves the protein synthesis ability. Based on the above, it was understood that it is preferable to include a poly A and also a polynucleotide of a certain length, for example, from 10 to 40 bases, as an enhancer or stabilizer.

### SEQUENCE LISTING FREE TEXT

SEQ ID NOS: 1-6, 26: artificial 3' UTR
SEQ ID NOS: 7-25, 27-74: primer

## Claims

1. A translation enhancer to be used for a cell-free protein synthesis system,
wherein the translation enhancer comprises a nucleic acid of at most 200 bases in length as a 3' untranslated region linked adjacent to the 3' end of a coding region that encodes the amino acid sequence of a desired protein.

2. The translation enhancer of Claim 1, wherein the 3' untranslated region is at most 100 bases in length.

3. The translation enhancer of Claim 2, wherein the 3' untranslated region is at most 80 bases in length.

4. The translation enhancer of Claim 3, wherein the 3' untranslated region is at most 60 bases in length.

5. The translation enhancer of Claim 4, wherein the 3' untranslated region is at most 40 bases in length.

6. The translation enhancer of any of Claims 1-5, wherein the 3' untranslated region has a poly A sequence of two or more consecutive A.

7. The translation enhancer of Claim 6, wherein the 3' untranslated region has a poly A sequence of from five to 40 consecutive A.

8. The translation enhancer of Claim 6, wherein the 3' untranslated region has a poly A sequence of from five to 20 consecutive A, and the total length is at most 50.

9. The translation enhancer of any of Claims 1-8, wherein the 3' untranslated region is a base sequence represented by SEQ ID NO: 6 or 26, a base sequence in which from one to five bases have been substituted, added, inserted, or deleted in the said sequences, or a base sequence having identity of at least 85% with a base sequence represented by SEQ ID NO: 6 or 26, and has a base sequence having 3' untranslated region activity.

10. The translation enhancer according to any of Claims 1-9, wherein the desired protein is a fusion protein provided with a protein tag at the C end of an arbitrary protein.

11. The translation enhancer according to any of Claims 1-9, wherein the desired protein is a fusion protein provided with a protein tag at the N end of an arbitrary protein.

12. The translation enhancer of any of Claims 1-11, which is a translation enhancer for use in a cell-free protein synthesis system including wheat germ extract.

13. An expression vector to be used for a cell-free protein synthesis system,
wherein the vector carries a 3' untranslated region of at most 200 bases in length on the 3' side of the insertion site of a coding region that encodes the amino acid sequence of a desired protein.

14. The vector of Claim 13, wherein the 3' untranslated region carries a region that encodes a protein tag on the 5' side thereof.

15. A template nucleic acid to be used for a cell-free protein synthesis system,
wherein the template nucleic acid comprises
a promoter region,
a coding region that encodes the amino acid sequence of a desired protein linked operably by the promoter region,
and a 3' untranslated region of the coding region, comprising the translation enhancer of any of Claims 1-12.

16. The template nucleic acid of Claim 15, wherein the coding region is a region that encodes a fusion protein provided with a protein tag at the C end of an arbitrary protein as the desired protein.

17. The template nucleic acid of Claim 15 or 16, wherein the coding region is a region that encodes a fusion protein provided with a protein tag at the N end of an arbitrary protein as the desired protein.

18. The template nucleic acid of any of Claims 15-17, wherein the template nucleic acid is transcription template DNA.

19. The template nucleic acid of any of Claims 15-17, wherein the template nucleic acid is translation template mRNA.

20. A primer set to be used for DNA polymerase amplification to synthesize transcription template DNA for a cell-free protein synthesis system,
wherein the primer set comprises
one or more forward primers,
one or more reverse primers,
and the one or more reverse primers are provided with a translation enhancer of any of Claims 1-12, or a part thereof.

21. The primer set of Claim 20, wherein the one or more reverse primers are also provided with a C end protein tag coding region adjacent to the 3' end side of the translation enhancer.

22. The primer set of Claim 20 or 21, wherein the one or more forward primers have an N end protein tag coding region.

23. A cell-free protein synthesis kit comprising:
a primer set of any of Claims 20-22,
and a medium for cell-free protein synthesis.

24. The kit of Claim 23, wherein the medium for cell-free protein synthesis is derived from wheat germ.

25. A method for producing transcription template DNA to be used for a cell-free protein synthesis system,
wherein the method comprises
a step for synthesizing transcription template DNA by carrying out a nucleic acid amplification reaction on DNA including a coding region of a desired protein using a primer set of any of Claims 20-22.

26. A method for producing a translation template to be used for a cell-free protein synthesis system,
wherein the method comprises
a step for synthesizing translation template mRNA using the template nucleic acid of any of Claims 15-18 in the absence of cells and in the presence of elements for transcribing transcription template DNA into mRNA.

27. A method for producing a protein,
wherein the method comprises
a step for synthesizing a protein using a template nucleic acid of Claim 19 in the absence of cells and in the presence of elements for translating translation template mRNA into a protein.

28. An array of translation template DNA,
wherein the array carries a plurality of template nucleic acids of any of Claims 15-18 corresponding to a plurality of proteins as transcription template DNA.

29. An RNA stabilizer,
wherein the RNA stabilizer comprises a nucleic acid of at most 200 bases in length as a 3' untranslated region linked adjacent to the 3' end of a coding region that encodes the amino acid sequence of a desired protein.
